# EUROPEAN PATENT SPECIFICATION

(11) **EP 1 694 627 B1**
(45) Date of publication and mention of the grant of the patent: **27.05.2009**
(21) Application number: 04804395.4
(22) Date of filing: 07.12.2004
(51) Int. Cl.: C07C 65/28, C07C 69/94, C07C 69/007, C07C 69/76, C07C 69/68, C07C 235/42, C07C 317/22, C07D 333/40, A61K 8/18, A61P 17/00, A61K 31/381, A61K 31/435, A61K 31/192, A61K 31/235

(54) **NOVEL LIGANDS THAT ARE ACTIVATORS OF THE RAR RECEPTORS, USE IN HUMAN MEDICINE AND IN COSMETICS**
NEUE LIGANDEN, BEI DENEN ES SICH UM AKTIVATOREN DES RAR-REZEPTORS HANDELT, VERWENDUNG IN DER HUMANMEDIZIN UND IN KOSMETIKA
NOUVEAUX LIGANDS ACTIVATEURS DES RECEPTEURS RAR S'UTILISANT EN MEDECINE ET EN COSMETIQUE

(30) Priority: 08.12.2003 FR 0314336; 17.12.2003 US 529986 P
(43) Date of publication of application: 30.08.2006
(73) Proprietor: GALDERMA RESEARCH & DEVELOPMENT, 06410 Biot (FR)
(72) Inventor: BIADATTI, Thibaud, F-06650 Opio (FR); ARLABOSSE, Jean.Marie, 06800 Cagnes sur Mer (FR)
(74) Representative: Allab, Myriam
(86) International application number: PCT/EP2004/014809
(87) International publication number: WO 2005/056510

(56) References cited:
- US-A- 5 716 624

## Description

The invention relates to novel compounds as novel and useful industrial products, which compounds are ligands that are activators of the RAR receptors. The invention also relates to compositions containing them and to their use in pharmaceutical compositions for use in human or veterinary medicine, or alternatively in cosmetic compositions and to the non-therapeutic use of these compositions.

Compounds with activity of retinoid type (vitamin A and its derivatives) are widely described in the literature as having activity in cell proliferation and differentiation processes. These properties give this class of compounds high potential in the treatment or prevention of numerous pathologies, and more particularly in dermatology and cancer. Many biological effects of retinoids are mediated by modulating the nuclear retinoic acid receptors (RAR).

The RAR receptors activate transcription by binding to DNA sequence elements, known as RAR response elements (RARE), in the form of a heterodimer with the retinoid X receptors (known as RXRs).

Three subtypes of human RARs have been identified and described: RARα, RARβ and RARγ.

Chemical compounds with activating activity on receptors of RAR type are known from the prior art. Mention may be made especially of the aromatic heterocyclic biaryl compounds described in patent EP 0 816 352 B1, which find applications in the treatment of dermatological, rheumatic, respiratory and ophthalmological complaints and also in the cosmetics field.

Patent EP 0 661 258 protects biaromatic propynyl compounds more particularly containing an alkoxy substituent on the aromatic ring. This alkyl chain limits the solubility of the compounds.

It thus appears to be advantageous to have available compounds with better solubility in water, so as to have compounds with physicochemical and pharmacokinetic properties that are more suitable for application as medicinal products or cosmetics for man; this better solubility would also allow greater ease of formulation of the active principle in a composition.

The Applicant has now discovered, surprisingly and unexpectedly, novel compounds that are retinoic acid receptor-activating ligands, containing an O-substituted hydroxyalkyl radical on the aromatic ring, allowing greater solubility in water, and which find applications in human medicine, especially in dermatology, and in the cosmetics field.

Thus, the present invention relates to compounds corresponding to the following general formula (I) below: 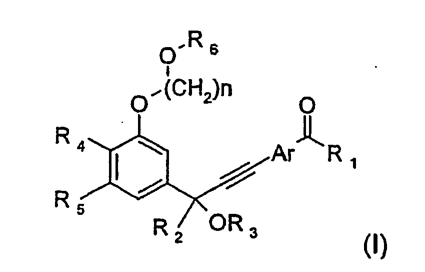 in which:
Ar represents a radical chosen from the radicals of formulae (a)-(c) below: 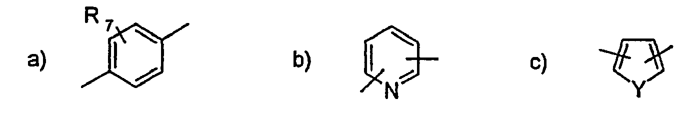
R₇ and Y having the meanings given below,

- R₁ represents a radical:
   (a) a hydrogen atom,
   (b) an alkyl radical containing from 1 to 6 carbon atoms,
   (c) a radical of formula: 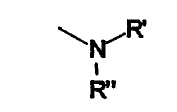
   (d) a radical -OR₈;
   R, R" and R₈ having the meanings given below,
- R₂ represents a hydrogen atom or an alkyl radical of 1 to 6 carbon atoms,
- R₃ represents a hydrogen atom, an alkyl radical containing from 1 to 6 carbon atoms or a radical -CO-R₉;
   R₉ having the meaning given below,
- R₄ and R₅, taken together, can form, with the adjacent aromatic ring, a 5- or 6-membered ring optionally substituted with methyl groups and/or optionally interrupted with an oxygen or sulfur atom,
- R₆ represents an alkyl radical containing from 1 to 6 carbon atoms or a radical (CO)R₁₀;
   R₁₀ having the meanings given below,
- R₇ represents a hydrogen atom, a halogen atom, an alkyl radical containing from 1 to 6 carbon atoms or a radical -OR₁₁,
   R₁₁ having the meaning given below,
- R₈ represents a hydrogen atom, a linear or branched alkyl radical containing from 1 to 20 carbon atoms, an alkenyl radical, a mono- or polyhydroxyalkyl radical, an optionally substituted aryl or aralkyl radical or a sugar residue or an amino acid or peptide residue,
- R₉ and R₁₀ represent CF₃ or an alkyl radical containing from 1 to 6 carbon atoms;
- R₁₁ represents H, an alkyl radical containing from 1 to 6 carbon atoms or a radical (CO) -R₁₂;
   R₁₂ having the meaning given below,
- R₁₂ represents an alkyl radical containing from 1 to 6 carbon atoms;
- R' and R" represent a hydrogen atom, an alkyl radical containing from 1 to 6 carbon atoms, a mono- or polyhydroxyalkyl radical, an optionally substituted aryl radical or an amino acid, peptide or sugar residue, or alternatively, taken together, form a heterocycle,
- Y represents an oxygen or sulfur atom;
- n is an integer between 2 and 6,
and the salts of the compounds of formula (I) when R₁ represents an OH function, and also the optical and geometrical isomers of the said compounds of formula (I).

When the compounds according to the invention are in the form of a salt, it is preferably an alkali metal or alkaline-earth metal salt, or alternatively a zinc salt or a salt of an organic amine.

According to the present invention, the term "alkyl containing from 1 to 6 carbon atoms" preferably means methyl, ethyl, n-propyl, i-propyl, c-propyl, isopropyl, n-butyl, i-butyl, t-butyl, n-pentyl or n-hexyl radicals.

The term "linear or branched alkyl containing from 1 to 20 carbon atoms" especially means methyl, ethyl, n-propyl, i-propyl, c-propyl, isopropyl, cyclopropyl, n-butyl, i-butyl, t-butyl, n-pentyl, n-hexyl, 2-ethylhexyl, octyl, dodecyl, hexadecyl or octadecyl radicals.

The term "monohydroxyalkyl radical" means a radical preferably containing 2 or 3 carbon atoms, especially a 2-hydroxyethyl, 2-hydroxypropyl or 3-hydroxypropyl radical.

The term "polyhydroxyalkyl radical" means a radical preferably containing from 3 to 6 carbon atoms and from 2 to 5 hydroxyl groups, such as 2,3-dihydroxypropyl, 2,3,4-trihydroxybutyl or 2,3,4,5-tetrahydroxypentyl radicals or a pentaerythritol residue.

The term "aryl radical" preferably means a phenyl radical optionally substituted with at least one halogen, a hydroxyl or a nitro function.

The term "aralkyl radical" preferably means a benzyl or phenethyl radical optionally substituted with at least one halogen, a hydroxyl or a nitro function.

The term "alkenyl radical" means a radical preferably containing from 1 to 5 carbon atoms and containing one or more ethylenic unsaturations, more particularly such as an allyl radical.

The term "sugar residue" means a residue derived especially from glucose, galactose or mannose, or alternatively from glucuronic acid, such as 6'-mannosyl, 6'-glucosyl or 6'-galactosyl.

The term "amino acid residue" especially means a residue derived from lysine, glycine or aspartic acid, and the term "peptide residue" more particularly means a dipeptide or tripeptide residue resulting from the combination of amino acids.

The term "heterocycle" preferably means a piperidino, morpholino, pyrrolidino or piperazino radical, optionally substituted in position 4 with a C₁-C₆ alkyl radical or a mono- or polyhydroxyalkyl radical as defined above.

The term "halogen atom" preferably means a fluorine, chlorine or bromine atom.

According to the present invention, the compounds of formula (I) that are more particularly preferred are those for which at least one, and preferably all, of the conditions below are met:
Ar represents the radical (a),
n is 2 or 3,
R₂ represents a hydrogen atom,
R₄ and R₅ form, with the adjacent aromatic ring, a 6-membered ring,
R₆ represents an alkyl radical.

Among the compounds of formula (I) falling within the context of the present invention, mention may be made especially of the following compounds:
1. 4-{3-Hydroxy-3-[4-(2-methoxyethoxy)-5,5,8,8-tetramethyl-5,6,7,8-tetrahydronaphth-2-yl]prop-1-ynyl}benzoic acid
2. 4-{3-Hydroxy-3-[4-(2-ethoxyethoxy)-5,5,8,8-tetramethyl-5,6,7,8-tetrahydronaphth-2-yl]prop-1-ynyl}benzoic acid
3. 4-{3-Hydroxy-3-[4-(2-ethoxyethoxy)-5,5,8,8-tetramethyl-5,6,7,8-tetrahydronaphth-2-yl]prop-1-ynyl}-2-hydroxybenzoic acid
4. 4-{3-Hydroxy-3-[4-(2-ethoxyethoxy)-5,5,8,8-tetramethyl-5,6,7,8-tetrahydronaphth-2-yl]prop-1-ynyl}benzoic (S)-acid
5. 4-{3-Hydroxy-3-[4-(2-ethoxyethoxy)-5,5,8,8-tetramethyl-5,6,7,8-tetrahydronaphth-2-yl]prop-1-ynyl}benzoic (R)-acid
6. 6-{3-Hydroxy-3-[4-(2-ethoxyethoxy)-5,5,8,8-tetramethyl-5,6,7,8-tetrahydronaphth-2-yl]prop-1-ynyl}nicotinic acid
7. 5-{3-Hydroxy-3-[4-(2-ethoxyethoxy)-5,5,8,8-tetramethyl-5,6,7,8-tetrahydronaphth-2-yl]prop-1-ynyl}pyridine-2-carboxylic acid
8. 5-{3-[4-(2-Ethoxyethoxy)-5,5,8,8-tetramethyl-5,6,7,8-tetrahydronaphth-2-yl]-3-hydroxyprop-1 ynyl}thiophene-2-carboxylic acid
9. 4-{3-[4-(2-Ethoxyethoxy)-5,5,8,8-tetramethyl-5,6,7,8-tetrahydronaphth-2-yl]-3-hydroxyprop-1-ynyl}thiophene-2-carboxylic acid
10. 5-{3-[4-(2-Ethoxyethoxy)-5,5,8,8-tetramethyl-5,6,7,8-tetrahydronaphth-2-yl]-3-hydroxyprop-1-ynyl}thiophene-3-carboxylic acid
11. 5-{3-[4-(2-Ethoxyethoxy)-5,5,8,8-tetramethyl-5,6,7,8-tetrahydronaphth-2-yl]-3-hydroxyprop-1-ynyl}furan-2-carboxylic acid
12. 4-{3-[4-(3-Ethoxyethoxy)-5,5,8,8-tetramethyl-5,6,7,8-tetrahydronaphth-2-yl]-3-hydroxyprop-1-ynyl}benzoic acid
13. 4-{3-[4-(3-Methoxypropoxy)-5,5,8,8-tetramethyl-5,6,7,8-tetrahydronaphth-2-yl]-3-hydroxyprop-1-ynyl}benzoic acid
14. 4-{3-[4-(4-Methoxybutoxy)-5,5,8,8-tetramethyl-5,6,7,8-tetrahydronaphth-2-yl]-3-hydroxyprop-1-ynyl}benzoic acid
15. 4-{3-[4-(5-Methoxypentoxy)-5,5,8,8-tetramethyl-5,6,7,8-tetrahydronaphth-2-yl]-3-hydroxyprop-1-ynyl}benzoic acid
16. 4-{3-[4-(6-Methoxyhexyloxy)-5,5,8,8-tetramethyl-5,6,7,8-tetrahydronaphth-2-yl]-3-hydroxyprop-1-ynyl}benzoic acid
17. 4-{3-[4-(2-Ethoxyethoxy)-5,5,8,8-tetramethyl-5,6,7,8-tetrahydronaphth-2-yl]-3-hydroxybut-1-ynyl}benzoic acid
18. 4-{3-[4-(2-Ethoxyethoxy)-5,5,8,8-tetramethyl-5,6,7,8-tetrahydronaphth-2-yl]-3-hydroxypent-1-ynyl}benzoic acid
19. 4-{3-[4-(2-Ethoxyethoxy)-5,5,8,8-tetramethyl-5,6,7,8-tetrahydronaphth-2-yl]-3-acetoxyprop-1-ynyl}benzoic acid
20. 4-{3-[4-(2-Ethoxyethoxy)-5,5,8,8-tetramethyl-5,6,7,8-tetrahydronaphth-2-yl]-3-propionyloxyprop-1-ynyl}benzoic acid
21. 4-{3-[7-(2-Ethoxyethoxy)-1,1,3,3-tetramethyl-1,3-dihydroisobenzofuran-5-yl]-3-hydroxyprop-1-ynyl}benzoic acid
22. 4-{3-[8-(2-Ethoxyethoxy)-4,4-dimethylchroman-6-yl]-3-hydroxyprop-1-ynyl}benzoic acid
23. 4-{3-[8-(2-Ethoxyethoxy)-4,4-dimethylthiochroman-6-yl]-3-hydroxyprop-1-ynyl}benzoic acid
24. 4-{3-[5-(2-Ethoxyethoxy)-4,4-dimethylchroman-7-yl]-3-hydroxyprop-1-ynyl}benzoic acid
25. 4-{3-[5-(2-Ethoxyethoxy)-4,4-dimethylthiochroman-7-yl]-3-hydroxyprop-1-ynyl}benzoic acid
26. 4-{3-[4-(2-Acetoxyethoxy)-5,5,8,8-tetramethyl-5,6,7,8-tetrahydronaphth-2-yl]-3-hydroxyprop-1-ynyl}benzoic acid
27. 4-(3-Hydroxy-3-{5,5,8,8-tetramethyl-4-[2-(2,2,2-trifluoroacetoxy)ethoxy]-5,6,7,8-tetrahydronaphth-2-yl}prop-1-ynyl)benzoic acid
28. 4-(3-Hydroxy-3-[5,5,8,8-tetramethyl-4-(2-propionyloxyethoxy)-5,6,7,8-tetrahydronaphth-2-yl]prop-1-ynyl}benzoic acid
29. 4-{3-[4-(2-Ethoxyethoxy)-5,5,8,8-tetramethyl-5,6,7,8-tetrahydronaphth-2-yl]-3-hydroxyprop-1-ynyl}-2-methylbenzoic acid
30. 2-Chloro-4-{3-[4-(2-ethoxyethoxy)-5,5,8,8-tetramethyl-5,6,7,8-tetrahydronaphth-2-yl]-3-hydroxyprop-1-ynyl}benzoic acid
31. 4-{3-[4-(2-Ethoxyethoxy)-5,5,8,8-tetramethyl-5,6,7,8-tetrahydronaphth-2-yl]-3-hydroxyprop-1-ynyl}-2-fluorobenzoic acid
32. 4-{3-[4-(2-Ethoxyethoxy)-5,5,8,8-tetramethyl-5,6,7,8-tetrahydronaphth-2-yl]-3-hydroxyprop-1-ynyl}-3-fluorobenzoic acid
33. 4-{3-[4-(2-Ethoxyethoxy)-5,5,8,8-tetramethyl-5,6,7,8-tetrahydronaphth-2-yl]-3-hydroxyprop-1-ynyl}-3-methylbenzoic acid
34. Ethyl 4-{3-[4-(2-ethoxyethoxy)-5,5,8,8-tetramethyl-5,6,7,8-tetrahydronaphth-2-yl]-3-hydroxyprop-1-ynyl}benzoate
35. Isopropyl 4-{3-[4-(2-ethoxyethoxy)-5,5,8,8-tetramethyl-5,6,7,8-tetrahydronaphth-2-yl]-3-hydroxyprop-1-ynyl}benzoate
36. Isobutyl 4-{3-[4-(2-ethoxyethoxy)-5,5,8,8-tetramethyl-5,6,7,8-tetrahydronaphth-2-yl]-3-hydroxyprop-1-ynyl}benzoate
37. 2,3-Dihydroxypropyl 4-{3-[4-(2-ethoxyethoxy)-5,5,8,8-tetramethyl-5,6,7,8-tetrahydronaphth-2-yl]-3-hydroxyprop-1-ynyl}benzoate
38. 6-Glucosyl 4-{3-[4-(2-ethoxyethoxy)-5,5,8,8-tetramethyl-5,6,7,8-tetrahydronaphth-2-yl]-3-hydroxyprop-1-ynyl}benzoate
39. 6-Galactosyl 4-{3-[4-(2-ethoxyethoxy)-5,5,8,8-tetramethyl-5,6,7,8-tetrahydronaphth-2-yl]-3-hydroxyprop-1-ynyl}benzoate
40. 4-{3-[4-(2-Ethoxyethoxy)-5,5,8,8-tetramethyl-5,6,7,8-tetrahydronaphth-2-yl]-3-hydroxyprop-1-ynyl}-N,N-dimethylbenzamide
41. 4-{3-[4-(2-Ethoxyethoxy)-5,5,8,8-tetramethyl-5,6,7,8-tetrahydronaphth-2-yl]-3-hydroxyprop-1-ynyl}-N,N-diethylbenzamide
42. (4-{3-[4-(2-Ethoxyethoxy)-5,5,8,8-tetramethyl-5,6,7,8-tetrahydronaphth-2-yl]-3-hydroxyprop-1-ynyl}phenyl)piperidin-1-ylmethanone
43. (4-{3-[4-(2-Ethoxyethoxy)-5,5,8,8-tetramethyl-5,6,7,8-tetrahydronaphth-2-yl]-3-hydroxyprop-1-ynyl}phenyl)morpholin-4-ylmethanone
44. (4-{3-[4-(2-Ethoxyethoxy)-5,5,8,8-tetramethyl-5,6,7,8-tetrahydronaphth-2-yl]-3-hydroxyprop-1-ynyl}phenyl)pyrrolidin-1-ylmethanone
45. (4-{3-[4-(2-Ethoxyethoxy)-5,5,8,8-tetramethyl-5,6,7,8-tetrahydronaphth-2-yl]-3-hydroxyprop-1-ynyl}-N-ethylbenzamide
46. (4-{3-[4-(2-Ethoxyethoxy)-5,5,8,8-tetramethyl-5,6,7,8-tetrahydronaphth-2-yl]-3-hydroxyprop-1-ynyl}benzoylamino)acetic acid
47. 2-(4-{3-[4-(2-Ethoxyethoxy)-5,5,8,8-tetramethyl-5,6,7,8-tetrahydronaphth-2-yl]-3-hydroxyprop-1-ynyl}benzoylamino)-3-phenylpropionic acid.

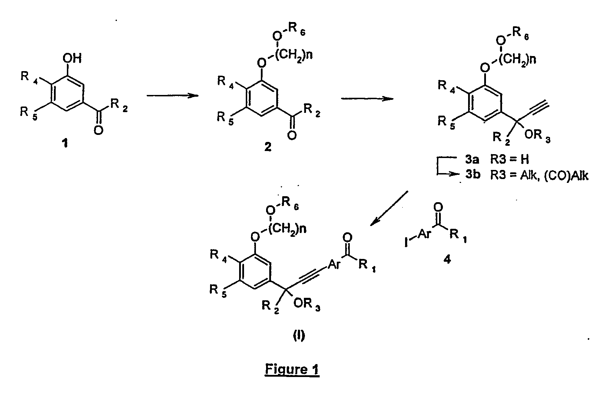

The subject of the present invention is also the processes for preparing the compounds of formula (I), in particular according to the reactions schemes given in Figure 1.

The compounds of formula (I) may be obtained from starting materials of general formula 1. Specifically, the intermediate of general formula 2 may be obtained after alkylation of the phenoxide derived from the compounds **1** in the presence, for example, of sodium hydride, with a corresponding alkylating group. The propargyl alcohol function of **3a** may be obtained by reaction with an ethynyl anion, for example in the presence of ethynylmagnesium bromide. The compounds of general formula **3b** may be obtained by simple alkylation or acylation reaction, for example by reaction with an alkyl iodide in the presence of silver oxide or an acyl chloride in basic medium, respectively. The compounds of general formula (**I**) are then obtained by coupling the compounds of formula **3** with a corresponding aryl iodide **4**, for example under the Sonogashira coupling conditions.

The compounds according to the invention have activating properties of RAR-type receptors. This RAR-receptor activating activity is measured in a test of transactivation by means of the dissociation constant Kdapp (apparent) and the AC₅₀ (concentration that gives 50% of the reference molecule activity).

According to the invention, the expression "activator of RAR-type receptors" means any compound which, for at least one of the RAR subtypes, has a dissociation constant Kdapp and an AC₅₀ value of less than or equal to 1 µm, in a transactivation test as described in Example 6.

The preferred compounds of the present invention have, for at least one of the RAR subtypes, a dissociation constant Kdapp of less than or equal to 500 nM and advantageously less than or equal to 100 Nm, and an AC₅₀ ≤ 100 nM.

A subject of the present invention is also the compounds of formula (I) as described above, as medicinal products.

The compounds according to the invention are particularly suitable in the following fields of treatment:
1) for treating dermatological complaints associated with a keratinization disorder relating to cell differentiation and proliferation, especially for treating common acne, comedones, polymorphs, acne rosacea, nodulocystic acne, acne conglobata, senile acne, and secondary acnes such as solar acne, medication-related acne or occupational acne;
2) for treating other types of keratinization disorders, especially ichthyosis, ichthyosiform conditions, Darier's disease, palmoplantar keratoderma, leukoplakia and leukoplakiform conditions, and cutaneous or mucous (buccal) lichen;
3) for treating other dermatological complaints with an inflammatory immunoallergic component, with or without cell proliferation disorder, and especially all forms of psoriasis, whether cutaneous, mucous or ungual, and even psoriatic rheumatism, or cutaneous atopy, such as eczema, or respiratory atopy, or alternatively gingival hypertrophy;
4) for treating all dermal or epidermal proliferations, whether benign or malignant, and whether of viral origin or otherwise, such as common warts, flat warts and verruciform epidermodysplasia, oral or florid papillomatoses, T lymphoma, and proliferations that may be induced by ultraviolet radiation, especially in the case of basocellular and spinocellular epithelioma, and also any cutaneous precancerous lesion such as keratoacanthomas;
5) for treating other dermatological disorders such as immune dermatoses, such as lupus erythematosus, immune bullous diseases and collagen diseases, such as scleroderma;
6) in the treatment of dermatological or general complaints with an immunological component;
7) for treating certain ophthalmological disorders, especially corneopathies,
8) for preventing or curing the stigmata of epidermal and/or dermal atrophy induced by local or systemic corticosteroids, or any other form of cutaneous atropy,
9) in the treatment of any cutaneous or general complaint of viral origin,
10) in the treatment of skin disorders caused by exposure to UV radiation, and also for repairing or combating ageing of the skin, whether photoinduced or chronological ageing, or for reducing pigmentations and actinic keratosis, or any pathology associated with chronological or actinic ageing, such as xerosis;
11) for combating sebaceous function disorders, such as the hyperseborrhoea of acne or simple seborrhoea;
12) for preventing or treating cicatrization disorders, or for preventing or repairing stretch marks, or alternatively for promoting cicatrization;
13) in the treatment of pigmentation disorders, such as hyperpigmentation, melasma, hypopigmentation or vitiligo;
14) in the treatment of lipid metabolism complaints, such as obesity, hyperlipidaemia, or non-insulin-dependent diabetes;
15) in the treatment of inflammatory complaints such as arthritis;
16) in the treatment or prevention of cancerous or precancerous conditions;
17) in the prevention or treatment of alopecia of various origins, especially alopecia caused by chemotherapy or radiation;
18) in the treatment of disorders of the immune system, such as asthma, type I sugar diabetes, multiple sclerosis or other selective dysfunctions of the immune system; and
19) in the treatment of complaints of the cardiovascular system, such as arteriosclerosis or hypertension.

A subject of the present invention is also a pharmaceutical composition comprising, in a physiologically acceptable medium, at least one compound of formula (I) as defined above.

A subject of the present invention is also a novel medicinal composition intended especially for treating the abovementioned complaints, which is characterized in that it comprises, in a pharmaceutically acceptable support that is compatible with the mode of administration selected for this composition, at least one compound of formula (I), an optical isomer thereof or a salt thereof.

The composition according to the invention may be administered orally, enterally, parenterally, topically or ocularly. The pharmaceutical composition is preferably packaged in a form that is suitable for topical application.

Via the oral route, the composition may be in the form of tablets, gel capsules, dragees, syrups, suspensions, solutions, powders, granules, emulsions, suspensions of microspheres or nanospheres or lipid or polymer vesicles allowing a controlled release. Via the parenteral route, the composition may be in the form of solutions or suspensions for infusion or for injection.

The compounds according to the invention are generally administered at a daily dose of about 0.01 mg/kg to 100 mg/kg of body weight, in 1 to 3 dosage intakes.

The compounds are used systemically, at a concentration generally of between 0.001% and 10% by weight and preferably between 0.01% and 1% by weight relative to the weight of the composition.

Via the topical route, the pharmaceutical composition according to the invention is more particularly intended for treating the skin and mucous membranes and may be in liquid, pasty or solid form, and more particularly in the form of ointments, creams, milks, pomades, powders, impregnated pads, syndets, solutions, gels, sprays, mousses, suspensions, sticks, shampoos or washing bases. It may also be in the form of suspensions of microspheres or nanospheres or of lipid or polymer vesicles or gelled or polymer patches allowing a controlled release.

The compounds are used topically at a concentration generally of between 0.001% and 10% by weight and preferably between 0.01% and 1% by weight, relative to the total weight of the composition.

The compounds of formula (I) according to the invention also find an application in cosmetics, in particular in body and hair hygiene and especially for treating acne-prone skin, for promoting regrowth of the hair or for limiting hair loss, for combating the greasy appearance of the skin or the hair, in protection against the harmful aspects of sunlight or in the treatment of physiologically dry skin, and for preventing and/or combating photoinduced or chronological ageing.

A subject of the invention is thus also a cosmetic composition comprising, in a physiologically acceptable support, at least one of the compounds of formula (I).

A subject of the invention is also the non-therapeutic use of a cosmetic composition comprising at least one compound of formula (I) for preventing and/or treating the signs of ageing and/or dry skin.

A subject of the invention is also the non-therapeutic use of a cosmetic composition comprising at least one compound of formula (I) for body or hair hygiene.

The cosmetic composition according to the invention containing, in a physiologically acceptable medium, at least one compound of formula (I) or an optical or geometrical isomer thereof or a salt thereof, may be especially in the form of a cream, a milk, a gel, suspensions of microspheres or nanospheres or lipid or polymer vesicles, impregnated pads, solutions, sprays, mousses, sticks, soaps, washing bases or shampoos.

The concentration of compound of formula (I) in the cosmetic composition is preferably between 0.001% and 3% by weight relative to the total weight of the composition.

The term "physiologically acceptable medium" means a medium that is compatible with the skin and possibly with its integuments (eyelashes, nails or hair) and/or mucous membranes.

The pharmaceutical and cosmetic compositions as described above may also contain inert additives, or even pharmacodynamically active additives as regards the pharmaceutical compositions, or combinations of these additives, and especially:
- wetting agents;
- flavour enhancers;
- preserving agents such as para-hydroxybenzoic acid esters;
- stabilizers;
- moisture regulators;
- pH regulators;
- osmotic pressure modifiers;
- emulsifiers;
- UV-A and UV-B screening agents;
- antioxidants such as α-tocopherol, butylhydroxyanisole, butylhydroxytoluene, superoxide dismutase, ubiquinol or certain metal-chelating agents;
- depigmenting agents such as hydroquinone, azelaic acid, caffeic acid or kojic acid;
- emollients;
- moisturizers, for instance glycerol, PEG 400, thiamorpholinone and its derivatives or urea;
- antiseborrhoeic or antiacne agents, such as S-carboxymethylcysteine, S-benzylcysteamine, salts thereof or derivatives thereof, or benzoyl peroxide;
- antibiotics, for instance erythromycin and its esters, neomycin, clindamycin and its esters, and tetracyclines;
- antifungal agents such as ketoconazole or poly-4,5-methylene-3-isothiazolidones;
- agents for promoting regrowth of the hair, for instance Minoxidil (2,4-diamino-6-piperidinopyrimidine 3-oxide) and its derivatives, Diazoxide (7-chloro 3-methyl-1,2,4-benzothiadiazine 1,1-dioxide) and Phenytoin (5,4-diphenylimidazolidine-2,4-dione);
- non-steroidal anti-inflammatory agents;
- carotenoids and especially β-carotene;
- anti-psoriatic agents such as anthralin and its derivatives;
- eicosa-5,8,11,14-tetraynoic acid and eicosa-5,8,11-triynoic acid, and esters and amides thereof;
- retinoids, i.e. natural or synthetic RXR receptor ligands;
- corticosteroids or oestrogens;
- α-hydroxy acids and α-keto acids or derivatives thereof, such as lactic acid, malic acid, citric acid, glycolic acid, mandelic acid, tartaric acid, glyceric acid or ascorbic acid, and also salts, amides or esters thereof, or β-hydroxy acids or derivatives thereof, such as salicylic acid and its salts, amides or esters;
- ion-channel blockers such as potassium-channel blockers;
- or alternatively, more particularly for pharmaceutical compositions, in combination with medicinal products known to interfere with the immune system (for example cyclosporin, FK 506, glucocorticoids, monoclonal antibodies, cytokines or growth factors, etc.).

Needless to say, a person skilled in the art will take care to select the optional compound(s) to be added to these compositions such that the advantageous properties intrinsically attached to the present invention are not, or are not substantially, adversely affected by the envisaged addition.

Another subject of the invention relates to a cosmetic process for enhancing the skin, **characterized in that** a composition comprising at least one compound of formula (I) as defined above is applied to the skin.

Activation of the retinoic acid receptors with the compounds of formula (I) according to the invention makes it possible to obtain skin whose surface aspect is enhanced.

Several examples of the production of active compounds of formula (I) according to the invention, biological activity results and also various concrete formulations based on such compounds, will now be given, for illustrative purposes and with no limiting nature.

### EXAMPLES

### Example 1: 4-{3-Hydroxy-3-[4-(2-methoxyethoxy)-5,5,8,8-tetramethyl-5,6,7,8-tetrahydronaphth-2-yl]prop-1-ynyl}-benzoic acid

### a. 3-Bromo-5,5,8,8-tetramethyl-5,6,7,8-tetrahydronaphth-1-ol

60 g (347 mmol) of 3-bromophenol are dissolved in 600 mL of dichloromethane. This solution is added to a solution of 46 g (347 mmol) of aluminium chloride in 200 mL of dichloromethane. 127 g (694 mmol) of 2,5-dichloro-2,5-dimethylhexane are added in 10 g portions every 40 minutes. The medium is then stirred for 10 hours, after which it is poured onto ice and extracted with dichloromethane. The residue obtained is dissolved in ethyl ether and this organic phase is then washed with 1N sodium hydroxide solution, and then with water. The residue obtained is purified by chromatography (eluent: heptane and then 1/1 heptane/dichloromethane). A thick oil is obtained (67 g; yield = 68%).

### b. 4-Hydroxy-5,5,8,8-tetramethyl-5,6,7,8-tetrahydronaphthalene-2-carbaldehyde

30 g (106 mmol) of 3-bromo-5,5,8,8-tetramethyl-5,6,7,8-tetrahydronaphth-1-ol are dissolved in 500 mL of anhydrous THF. The medium is cooled to -78°C and 156 mL (265 mmol) of tert-butyllithium are then added dropwise. After 45 minutes at this temperature, 12.3 mL (159 mmol) of dimethylformamide are added. The mixture is warmed to room temperature and then treated with 1N hydrochloric acid solution and extracted with ethyl acetate. The residue obtained is then purified by chromatography (eluent: 9/1 heptane/ethyl acetate). A white solid is obtained (m = 16.5 g; yield = 67%; m.p. = 144°C).

### c. 5,5,8,8-Tetramethyl-4-(2-methoxyethoxy)-5,6,7,8-tetrahydronaphthalene-2-carbaldehyde

34 g (148 mmol) of 4-hydroxy-5,5,8,8-tetramethyl-5,6,7,8-tetrahydronaphthalene-2-carbaldehyde are dissolved in 400 mL of anhydrous DMF. 7.2 g (178 mmol) of 60% sodium hydride are then added portionwise, and the reaction medium is stirred for 1 hour. 16.5 mL (178 mmol) of 2-methoxyethyl chloride are added dropwise, and the medium is stirred at room temperature for 2 hours and then hydrolysed and extracted with ethyl ether. The organic phase is washed with 1N sodium hydroxide solution and then three times with water. The residue obtained is purified by chromatography (eluent: heptane). A yellow oil is obtained (m = 42.7 g; yield = 95%).

### d. 1- [5, 5, 8, 8-Tetramethyl-4- (2-methoxyethoxy) - 5,6,7,8-tetrahydronaphth-2-yl]prop-2-ynyl-1-ol

0.65 g (2.1 mmol) of 5,5,8,8-tetramethyl-4-(2-methoxyethoxy)-5,6,7,8-tetrahydronaphthalene-2-carbaldehyde is dissolved in 20 mL of THF. 6.4 mL (3.2 mmol) of 0.5N ethylmagnesium bromide solution are added and the reaction medium is then stirred for 1 hour. After treatment with 1N hydrochloric acid solution and extraction with ethyl acetate, followed by evaporation, the residue is purified by chromatography on a column of silica (eluent: 8/2 heptane/EtOAC). A yellow oil is obtained (m = 0.48 g; yield = 69%).

### e. 4-{3-Hydroxy-3-[4-(2-methoxyethoxy)-5,5,8,8-tetramethyl-5, 6, 7, 8-tetrahydronaphth-2-yl]prop-1-ynyl}-benzoic acid

0.4 g (1.2 mmol) of 1-[5,5,8,8-tetramethyl-4-(2-methoxyethoxy)-5,6,7,8-tetrahydronaphth-2-yl]prop-2-ynyl-1-ol and 210 mg (0.8 mmol) of 4-iodobenzoic acid are dissolved in 5 mL of DMF and 2 mL of triethylamine. 10 mg of copper iodide and 20 mg of dichloropalladium-bis(triphenylphosphine) are added and the reaction medium is stirred at 50°C for 2 hours 30 minutes. The reaction medium is poured into ammonium chloride solution and extracted with ethyl acetate. The residue is purified on a column of silica (eluent: 6/4 heptane/EtOAC). The desired product is obtained in the form of a yellowish powder (m = 290 mg; yield = 83%; m.p. = 150°C). ¹H NMR (DMSO D₆): 1.24 (s, 6H); 1.36 (s, 6H); 1.56-1.60 (m, 4H); 3.33 (s, 3H); 3.73 (m, 2H); 4.08 (m, 2H); 5.53 (d, J = 4.9 Hz, 1H); 6.10 (d, J = 4.8 Hz, 1H); 6.90 (s, 1H); 7.12 (s, 1H); 7.55 (d, J = 8.3 Hz, 2H); 7.93 (d, J = 8.3 Hz, 2H), 13.1 (bs, 1H).

### Example 2: 4-{3-Hydroxy-3-[4-(2-ethoxyethoxy)-5,5,8,8-tetramethyl-5,6,7,8-tetrahydronaphth-2-yl]prop-1-ynyl}-benzoic acid

### a. 5,5, 8, 8-Tetramethyl-4-(2-ethoxyethoxy)-5,6,7,8-tetrahydronaphthalene-2-carbaldehyde

36 g (156 mmol) of 4-hydroxy-5,5,8,8-tetramethyl-5,6,7,8-tetrahydronaphthalene-2-carbaldehyde are dissolved in 400 mL of anhydrous DMF. 6.5 g (163 mmol) of 60% sodium hydride are added portionwise and the reaction medium is stirred for 1 hour. 25 g (163 mmol) of 2-ethoxyethyl bromide are added portionwise and the medium is stirred at room temperature for 2 hours and then hydrolysed and extracted with ethyl ether. The organic phase is washed with 1N sodium hydroxide solution, and then three times with water. The residue obtained is purified by chromatography (eluent: heptane). A yellow oil is obtained (m = 40.7 g; yield = 86%).

### b. 1- [5, 5, 8, 8-Tetramethyl-4- (2-ethoxyethoxy)-5,6,7,8-tetrahydronaphth-2-yl]prbp-2-yn-1-ol

40 g (131 mmol) of 5,5,8,8-tetramethyl-4- (2-ethoxyethoxy)-5,6,7,8-tetrahydronaphthalene-2-carbaldehyde are dissolved in 400 mL of THF. 290 mL (145 mmol) of 0.5N ethynylmagnesium bromide solution are added and the reaction medium is then stirred for 1 hour. After treatment with 1N hydrochloric acid solution and extraction with ethyl acetate, followed by evaporation, the residue is purified by chromatography on a column of silica (eluent: 8/2 heptane/EtOAC). A yellow oil is obtained (m = 42 g; yield = 97%).

### c. 4-{3-Hydroxy-3-[4-(2-ethoxyethoxy)-5,5,8,8-tetramethyl-5,6,7,8-tetrahydronaphth-2-yl]-prop-1-ynyl}benzoic acid

5.4 g (15 mmol) of 1-[5,5,8,8-tetramethyl-4-(2-ethoxyethoxy)-5,6,7,8-tetrahydronaphth-2-yl]prop-2-yn-1-ol and 2.6 g (11 mmol) of 4-iodobenzoic acid are dissolved in 125 mL of DMF and 50 mL of triethylamine. 110 mg of copper iodide and 210 mg of dichloro-palladiumbis(triphenylphosphine) are added, and the reaction medium is stirred at 50°C for 2 hours 30 minutes. The reaction medium is poured into ammonium chloride solution and extracted with ethyl acetate. The residue is purified on a column of silica (eluent: 6/4 heptane/EtOAc). The desired product is obtained in the form of yellowish crystals (m = 2.5 g; yield = 50%; m.p. = 158°C) ¹H NMR (DMSO D₆): 1.12 (t, J = 7.0 Hz, 3H); 1.23 (s, 6H); 1.35 (s, 6H); 1.56-1.60 (m, 4H); 3.50 (q, J = 7.0 Hz, 2H); 3.75 (m, 2H); 4.06 (m, 2H); 5.52 (d, J = 5.0 Hz, 1H); 6.09 (d, J = 4.9 Hz, 1H); 6.90 (s, 1H); 7.11 (s, 1H); 7.54 (d, J = 8.3 Hz, 2H); 7.93 (d, J = 8.3 Hz, 2H), 13.1 (bs, 1H).

### Example 3: 4-{3-Hydroxy-3-[4-(2-ethoxyethoxy)-5,5,8,8-tetramethyl-5,6,7,8-tetrahydronaphth-2-yl]prop-1-ynyl}-2-hydroxybenzoic acid

### a. 4-{3-Hydroxy-3-[4-(2-ethoxyethoxy)-5,5,8,8-tetramethyl-5,6,7,8-tetrahydronaphth-2-yl]prop-1-ynyl}-2-hydroxybenzoic acid

200 mg (0.6 mmol) of 1-[5,5,8,8-tetramethyl-4-(2-ethoxyethoxy)-5,6,7,8-tetrahydronaphth-2-yl]prop-2-yn-1-ol (Example 2b) and 175 mg (0.4 mmol) of 2-hydroxy-4-iodobenzoic acid are dissolved in 5 mL of DMF and 2 mL of triethylamine. 10 mg of copper iodide and 20 mg of dichloropalladiumbis(triphenylphosphine) are added, and the reaction medium is stirred at 50°C for 2 hours 30 minutes. The reaction medium is poured into ammonium chloride solution and extracted with ethyl acetate. The residue is purified on a column of silica (eluent: 5/5 heptane/EtOAc). The desired product is obtained in the form of a yellowish powder (m = 80 mg; yield = 42%; m.p. = 120°C) ¹H NMR (DMSO D₆) : 1.13 (t, J = 7.0 Hz, 3H); 1.24 (s, 6H); 1.36 (s, 6H); 1.56-1.61 (m, 4H); 3.52 (q, J = 7.0 Hz, 2H); 3.76 (m, 2H); 4.07 (m, 2H); 5.51 (s, 1H); 6.15 (bs, 1H); 6.89 (s, 1H); 6.97 (m, 2H); 7.11 (s, 1H); 7.77-7.79 (m, 1H), 11.5 (bs, 1H).

### Example 4: 4-{3-Hydroxy-3-[4-(2-ethoxyethoxy)-5,5,8,8-tetramethyl-5,6,7,8-tetrahydronaphth-2-yl]-prop-1-ynyl}benzoic (S)-acid

### a. (R)-1-[5,5,8,8-Tetramethyl-4-(2-ethoxyethoxy)-5,6,7,8-tetrahydronaphth-2-yl]prop-2-yn-1-ol

36 g (110 mmol) of 1-[5,5,8,8-tetramethyl-4-(2-ethoxyethoxy)-5,6,7,8-tetrahydronaphth-2-yl]prop-2-yn-1-ol (Example 2b) are dissolved in 75 mL of heptane and 10.2 ml of vinyl acetate. 7.26 g of enzyme PS30 are added, and the reaction medium is stirred and heated to 38°C. After 72 hours, the reaction medium is filtered and concentrated under reduced pressure. Two products are separated out by chromatography: 1-[5,5,8,8-tetramethyl-4-(2-ethoxyethoxy)-5,6,7,8-tetrahydronaphth-2-yl]prop-2-yn-1-ol (R)-acetate and (S)-1-[5,5,8,8-tetramethyl-4-(2-ethoxyethoxy)-5,6,7,8-tetrahydronaphth-2-yl]prop-2-yn-1-ol, which will be used for Example 5. 18 g (48 mmol) of 1-[5,5,8,8-tetramethyl-4-(2-ethoxyethoxy)-5,6,7,8-tetrahydronaphth-2-yl]prop-2-yn-1-ol (R)-acetate are thus dissolved in 500 mL of a 2% solution of K₂CO₃ in methanol and the medium is stirred for 3 hours. The reaction medium is then treated with 0.01N hydrochloric acid solution and extracted with ethyl acetate. The residue obtained is purified by chromatography (eluent: 8/2 heptane/EtOAc). A colourless oil is obtained (m = 15 g, yield = 41%).

### b. 4-{3-Hydroxy-3-[4-(2-ethoxyethoxy)-5,5,8,8-tetramethyl-5,6,7,8-tetrahydronaphth-2-yl]prop-1-ynyl}-benzoic (S)-acid

In a manner similar to that of Example 2c, by reaction of 12 g (36 mmol) of (R)-1-[5,5,8,8-tetramethyl-4-(2-ethoxyethoxy)-5,6,7,8-tetrahydronaphth-2-yl]prop-2-yn-1-ol, 6.3 g (25 mmol) of 4-iodobenzoic acid in the presence of 275 mg of copper iodide and 500 mg of dichloropalladiumbis(triphenylphosphine). The desired product is obtained in the form of a whitish powder (m = 9.4 g; yield = 84%; m.p. = 96°C) ¹H NMR (DMSO D₆): 1.12 (t, J = 7.0 Hz, 3H); 1.23 (s, 6H); 1.35 (s, 6H); 1.56-1.60 (m, 4H); 3.50 (q, J = 7.0 Hz, 2H); 3.75 (m, 2H); 4.06 (m, 2H); 5.52 (d, J = 5.0 Hz, 1H); 6.09 (d, J = 4.9 Hz, 1H); 6.90 (s, 1H); 7.11 (s, 1H); 7.54 (d, J = 8.3 Hz, 2H); 7.93 (d, J = 8.3 Hz, 2H), 13.1 (bs, 1H).

### Example 5: 4-{3-Hydroxy-3-[4-(2-ethoxyethoxy)-5,5,8,8-tetramethyl-5,6,7,8-tetrahydronaphth-2-yl]prop-1-ynyl}-benzoic (R)-acid

In a manner similar to that of Example 4b, by reaction of 15 g (45 mmol) of (S)-1-[5,5,8,8-tetramethyl-4-(2-ethoxyethoxy)-5,6,7,8-tetrahydronaphth-2-yl]prop-2-yn-1-ol (obtained in Example 4a), 7.9 g (32 mmol) of 4-iodobenzoic acid in the presence of 350 mg of copper iodide and 630 mg of dichloro-palladiumbis(triphenylphosphine). The desired product is obtained in the form of a whitish powder (m = 8.7 g; yield = 47%; m.p. = 94°C) ¹H NMR (DMSO D₆): 1.12 (t, J = 7.0 Hz, 3H); 1.23 (s, 6H); 1.35 (s, 6H); 1.56-1.60 (m, 4H); 3.50 (q, J = 7.0 Hz, 2H); 3.75 (m, 2H); 4.06 (m, 2H); 5.52 (d, J = 5.0 Hz, 1H); 6.09 (d, J = 4.9 Hz, 1H); 6.90 (s, 1H); 7.11 (s, 1H); 7.54 (d, J = 8.3 Hz, 2H); 7.93 (d, J = 8.3 Hz, 2H), 13.1 (bs, 1H).

### EXAMPLE 6: TRANSACTIVATION TEST

The activation of receptors with an agonist (activator) in HeLa cells leads to the expression of a reporter gene, luciferase, which, in the presence of a substrate, generates light. The activation of the receptors may thus be measured by quantifying the luminescence produced after incubating the cells in the presence of a reference antagonist. The activating products displace the antagonist from its site, thus allowing activation of the receptor. The activity is measured by quantifying the increase in light produced. This measurement makes it possible to determine the activating activity of the compounds according to the invention.

### Determination of the Kdapp:

In this study, a constant is determined which represents the affinity of the molecule for the receptor. Since this value can fluctuate depending on the basal activity and the expression of the receptor, it is referred to as the Kdapparent (KdApp).

To determine this constant, the cells are brought into contact with a concentration of the test product and a concentration of the reference antagonist, 4-(5,5-dimethyl-8-p-tolyl-5,6-dihydronaphth-2-ylethynyl)benzoic acid. Measurements are also taken for the total agonist (4-[2-(5,5,8,8-tetramethyl-5,6,7,8-tetrahydronaphth-2-yl)propenyl]benzoic acid) and inverse agonist, 4-{(E)-3-[4-(4-tert-butylphenyl)-5,5,8,8-tetramethyl-5,6,7,8-tetrahydro-2-naphthyl]-3-oxopropenyl}benzoic acid, controls.

These crossed curves make it possible to determine the AC₅₀ values (concentration at which 50% activation is observed) for the reference ligand at various concentrations of test product. These AC₅₀ values are used to calculate the Schild regression by plotting a straight line corresponding to the Schild equation ("*quantitation in receptor pharmacology*" *Terry P.Kenakin, Receptors and Channels, 2001,7*,*371-385)*.

In the case of an agonist, an AC₅₀ value (concentration that gives 50% of the activity) is calculated by plotting the curve of the product at the concentration of the reference ligand that gives 80% activation.

The HeLa cell lines used are stable transfectants containing the plasmids ERE-βGlob-Luc-SV-Neo (reporter gene) and RAR (α, β, γ) ER-DBD-puro. These cells are inoculated into 96-well plates at a rate of 10 000 cells per well in 100 µl of DMEM medium without phenol red, and supplemented with 10% defatted calf serum. The plates are then incubated at 37°C and 7% CO₂ for 4 hours.

The various dilutions of the test products, of the reference ligand (4-(5,5-dimethyl-8-p-tolyl-5,6-dihydronaphth-2-yl)ethynyl)benzoic acid, of the 100% control (100 nM 4-[2-(5,5,8,8-tetramethyl-5,6,7,8-tetrahydro-2-naphthyl)propenyl]benzoic acid) and of the 0% control (500 nM 4-{(E)-3-[4-(4-tert-butylphenyl)-5,5,8,8-tetramethyl-5,6,7,8-tetrahydro-2-naphthyl]-3-oxopropenyl}benzoic acid) are added at a rate of 5 µl per well. The plates are then incubated for 18 hours at 37°C and 7% CO₂.

The culture medium is removed by turning over and 100 µl of a 1:1 PBS/luciferine mixture is added to each well. After 5 minutes, the plates are read using the luminescence detector.

| | **RAR alpha** | | **RAR beta** | | **RAR gamma** | |
|---|---|---|---|---|---|---|
| | **Kdapp (nM)** | **AC₅₀ (nM)** | **Kdapp (nM)** | **AC₅₀ (nM)** | **Kdapp (nM)** | **AC₅₀ (nM)** |
| **Ex 1** | 60 | 400 | 60 | 45 | 2 | 2 |
| **Ex 2** | 1000 | 900 | 250 | 500 | 30 | 50 |
| **Ex 3** | 30 | 150 | 120 | 100 | 1 | 1 |
| **Ex 4** | 120 | 800 | 250 | 200 | 1 | 1.2 |
| **Ex 5** | 120 | 800 | 250 | 200 | 1 | 1.8 |

The results obtained with the compounds according to the invention clearly show Kdapp values ≤ 100 nM and an AC₅₀ value ≤ 100 nM for at least one of the receptor subtypes, this clearly demonstrating an increase in the signal, and in the luminescence in the presence of the reference antagonist. The compounds according to the invention are thus clearly activators of retinoic acid receptors (RAR).

### EXAMPLE 7: FORMULATION EXAMPLES

This example illustrates various concrete formulations based on the compounds according to the invention.

### A - ORAL ROUTE

### (a) 0.2 g tablet

- Compound of Example 5 0.001 g
- Starch 0.114 g
- Dicalcium phosphate 0.020 g
- Silica 0.020 g
- Lactose 0.030 g
- Talc 0.010 g
- Magnesium stearate 0.005 g

### (b) Drinkable suspension in 5 ml ampoules

- Compound of Example 3 0.001 g
- Glycerol 0.500 g
- 70% sorbitol 0.500 g
- Sodium saccharinate 0.010 g
- Methyl para-hydroxybenzoate 0.040 g
- Flavouring qs
- Purified water qs 5 ml

### (c) 0.8 g tablet

- Compound of Example 4 0.500 g
- Pregelatinized starch 0.100 g
- Microcrystalline cellulose 0.115 g
- Lactose 0.075 g
- Magnesium stearate 0.010 g

### (d) Drinkable suspension in 10 ml ampoules

- Compound of Example 2 0.200 g
- Glycerol 1.000 g
- 70% sorbitol 1.000 g
- Sodium saccharinate 0.010 g
- Methyl para-hydroxybenzoate 0.080 g
- Flavouring qs
- Purified water qs 10 ml

### B - PARENTERAL ROUTE

### (a) Composition

- Compound of Example 3 0.002 g
- Ethyl oleate qs 10 g

### (b) Composition

- Compound of Example 1 0.05%
- Polyethylene glycol 20%
- 0.9% NaCl solution qs 100

### (c) Composition

- Compound of Example 3 2.5%
- Polyethylene glycol 400 20%
- 0.9% NaCl solution qs 100

### (d) Injectable cyclodextrin composition

- Compound of Example 3 0.1 mg
- β-Cyclodextrin 0.10 g
- Water for injection qs 10.00 g

### C - TOPICAL ROUTE

### (a) Ointment

- Compound of Example 2 0.020 g
- Isopropyl myristate 81.700 g
- Liquid petroleum jelly fluid 9.100 g
- Silica ("Aerosil 200" sold by Degussa) 9.180 g

### (b) Ointment

- Compound of Example 5 0.300 g
- White petroleum jelly codex qs 100 g

### (c) Nonionic water-in-oil cream

- Compound of Example 4 0.100 g
- Mixture of emulsifying lanolin alcohols, waxes and oils ("Anhydrous Eucerin" sold by BDF) 39.900 g
- Methyl para-hydroxybenzoate 0.075 g
- Propyl para-hydroxybenzoate 0.075 g
- Sterile demineralized water qs 100 g

### (d) Lotion

- Compound of Example 2 0.100 g
- Polyethylene glycol (PEG 400) 69.900 g
- 95% ethanol 30.000 g

### (e) Hydrophobic ointment

- Compound of Example 4 0.300 g
- Isopropyl myristate 36.400 g
- Silicone oil ("Rhodorsil 47.V 300" sold by Rhône-Poulenc) 36.400 g
- Beeswax 13.600 g
- Silicone oil ("Abil 300 000 cst" sold by Goldschmidt) qs 100 g

### (f) Nonionic oil-in-water cream

- Compound of Example 5 1.000 g
- Cetyl alcohol 4.000 g
- Glyceryl monostearate 2.500 g
- PEG-50 stearate 2.500 g
- Karite butter 9.200 g
- Propylene glycol 2.000 g
- Methyl para-hydroxybenzoate 0.075 g
- Propyl para-hydroxybenzoate 0.075 g
- Sterile demineralized water qs 100 g

## Claims

1. Compounds **characterized in that** they correspond to formula (I) below: 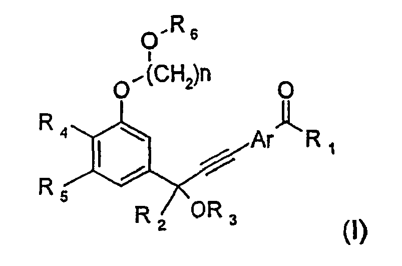 in which:
Ar represents a radical chosen from the radicals of formulae (a)-(c) below: 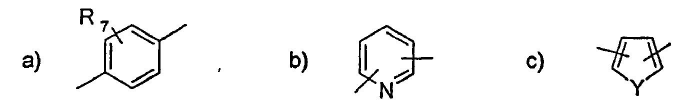
R₇ and Y having the meanings given below,
- R₁ represents a radical:
(a) a hydrogen atom,
(b) an alkyl radical containing from 1 to 6 carbon atoms,
(c) a radical of formula: 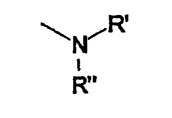
(d) a radical -OR₈;
R, R" and R₈ having the meanings given below,
- R₂ represents a hydrogen atom or an alkyl radical of 1 to 6 carbon atoms,
- R₃ represents a hydrogen atom, an alkyl radical containing from 1 to 6 carbon atoms or a radical -CO-R₉;
R₉ having the meaning given below,
- R₄ and R₅, taken together, can form, with the adjacent aromatic ring, a 5- or 6-membered ring optionally substituted with methyl groups and/or optionally interrupted with an oxygen or sulfur atom,
- R₆ represents an alkyl radical containing from 1 to 6 carbon atoms or a radical (CO)R₁₀;
R₁₀ having the meanings given below,
- R₇ represents a hydrogen atom, a halogen atom, an alkyl radical containing from 1 to 6 carbon atoms or a radical -OR₁₁,
R₁₁ having the meaning given below,
- R₈ represents a hydrogen atom, a linear or branched alkyl radical containing from 1 to 20 carbon atoms, an alkenyl radical, a mono- or polyhydroxyalkyl radical, an optionally substituted aryl or aralkyl radical or a sugar residue or an amino acid or peptide residue,
- R₉ and R₁₀ represent CF₃ or an alkyl radical containing from 1 to 6 carbon atoms;
- R₁₁ represents H, an alkyl radical containing from 1 to 6 carbon atoms or a radical (CO)-R₁₂;
R₁₂ having the meaning given below,
- R₁₂ represents an alkyl radical containing from 1 to 6 carbon atoms;
- R' and R" represent a hydrogen atom, an alkyl radical containing from 1 to 6 carbon atoms, a mono- or polyhydroxyalkyl radical, an optionally substituted aryl radical or an amino acid, peptide or sugar residue, or alternatively, taken together, form a heterocycle,
- Y represents an oxygen or sulfur atom;
- n is an integer between 2 and 6,
and the salts of the compounds of formula (I) when R₁ represents an OH function, and also the optical and geometrical isomers of the said compounds of formula (I).

2. Compounds according to Claim 1, **characterized in that** they are in the form of salts of an alkali metal or alkaline-earth metal, zinc salts, or salts of an organic amine.

3. Compounds according to either of Claims 1 and 2, **characterized in that** the alkyl radicals containing from 1 to 6 carbon atoms are chosen from the methyl, ethyl, n-propyl, i-propyl, c-propyl, isopropyl, n-butyl, i-butyl, t-butyl, n-pentyl and n-hexyl radicals.

4. Compounds according to one of Claims 1 to 3, **characterized in that** the alkyl radicals containing from 1 to 20 carbon atoms are chosen from methyl, ethyl, n-propyl, i-propyl, c-propyl, isopropyl, cyclopropyl, n-butyl, i-butyl, t-butyl, n-pentyl, n-hexyl, 2-ethylhexyl, octyl, dodecyl, hexadecyl and octadecyl radicals.

5. Compounds according to one of Claims 1 to 4, **characterized in that** the monohydroxyalkyl radicals are chosen from 2-hydroxyethyl, 2-hydroxypropyl and 3-hydroxypropyl radicals.

6. Compounds according to one of Claims 1 to 5, **characterized in that** the polyhydroxyalkyl radicals are chosen from 2,3-dihydroxypropyl, 2,3,4-trihydroxybutyl or 2,3,4,5-tetrahydroxypentyl radicals and a pentaerythritol residue.

7. Compounds according to any one of the preceding claims, **characterized in that** the aryl radicals are chosen from phenyl radicals optionally substituted with at least one halogen, a hydroxyl or a nitro function.

8. Compounds according to any one of the preceding claims, **characterized in that** the aralkyl radicals are chosen from benzyl and phenethyl radicals, optionally substituted with at least one halogen, a hydroxyl or a nitro function.

9. Compounds according to any one of the preceding claims, **characterized in that** the alkenyl radicals are chosen from radicals preferably containing 1 to 6 carbon atoms and containing one or more ethylenic unsaturations.

10. Compounds according to any one of the preceding claims, **characterized in that** the sugar residue is chosen from the group consisting of the residue derived from glucose, galactose or mannose, or alternatively from glucuronic acid.

11. Compounds according to any one of the preceding claims, **characterized in that** the amino acid residue is chosen from the group consisting of a residue derived from lysine, glycine or aspartic acid.

12. Compounds according to any one of the preceding claims, **characterized in that** the peptide residue is chosen from the group consisting of a dipeptide or tripeptide residue resulting from the combination of amino acids.

13. Compounds according to any one of the preceding claims, **characterized in that** the heterocyclic radicals are chosen from morpholino, pyrrolidino and piperazino radicals, optionally substituted in position 4 with a C₁-C₆ alkyl radical or a mono- or polyhydroxyalkyl radical.

14. Compounds according to any one of the preceding claims, **characterized in that** the halogen atoms are chosen from fluorine, chlorine and bromine atoms.

15. Compounds of formula (I) according to any one of the preceding claims, chosen from:
1. 4-{3-Hydroxy-3-[4-(2-methoxyethoxy)-5,5,8,8-tetramethyl-5,6,7,8-tetrahydronaphth-2-yl]prop-1-ynyl}benzoic acid
2. 4-{3-Hydroxy-3-[4-(2-ethoxyethoxy)-5,5,8,8-tetramethyl-5,6,7,8-tetrahydronaphth-2-yl]prop-1-ynyl}benzoic acid
3. 4-{3-Hydroxy-3-[4-(2-ethoxyethoxy)-5,5,8,8-tetramethyl-5,6,7,8-tetrahydronaphth-2-yl]prop-1-ynyl}-2-hydroxybenzoic acid
4. 4-{3-Hydroxy-3-[4-(2-ethoxyethoxy)-5,5,8,8-tetramethyl-5,6,7,8-tetrahydronaphth-2-yl]prop-1-ynyl}benzoic (S)-acid
5. 4-{3-Hydroxy-3-[4-(2-ethoxyethoxy)-5,5,8,8-tetramethyl-5,6,7,8-tetrahydronaphth-2-yl]prop-1-ynyl}benzoic (R)-acid
6. 6-{3-Hydroxy-3-[4-(2-ethoxyethoxy)-5,5,8,8-tetramethyl-5,6,7,8-tetrahydronaphth-2-yl]prop-1-ynyl}nicotinic acid
7. 5-{3-Hydroxy-3-[4-(2-ethoxyethoxy)-5,5,8,8-tetramethyl-5,6,7,8-tetrahydronaphth-2-yl]prop-1-ynyl}pyridine-2-carboxylic acid
8. 5-{3-[4-(2-Ethoxyethoxy)-5,5,8,8-tetramethyl-5,6,7,8-tetrahydronaphth-2-yl]-3-hydroxyprop-1-ynyl}thiophene-2-carboxylic acid
9. 4-{3-[4-(2-Ethoxyethoxy)-5,5,8,8-tetramethyl-5,6,7,8-tetrahydronaphth-2-yl]-3-hydroxyprop-1-ynyl}thiophene-2-carboxylic acid
10. 5-{3-[4-(2-Ethoxyethoxy)-5,5,8,8-tetramethyl-5,6,7,8-tetrahydronaphth-2-yl]-3-hydroxyprop-1-ynyl}thiophene-3-carboxylic acid
11. 5-{3-[4-(2-Ethoxyethoxy)-5,5,8,8-tetramethyl-5,6,7,8-tetrahydronaphth-2-yl]-3-hydroxyprop-1-ynyl}furan-2-carboxylic acid
12. 4-{3-[4-(3-Ethoxyethoxy)-5,5,8,8-tetramethyl-5,6,7,8-tetrahydronaphth-2-yl]-3-hydroxyprop-1-ynyl}benzoic acid
13. 4-{3-[4-(3-Methoxypropoxy)-5,5,8,8-tetramethyl-5,6,7,8-tetrahydronaphth-2-yl]-3-hydroxyprop-1-ynyl}benzoic acid
14. 4-{3-[4-(4-Methoxybutoxy)-5,5,8,8-tetramethyl-5,6,7,8-tetrahydronaphth-2-yl]-3-hydroxyprop-1-ynyl}benzoic acid
15. 4-{3-[4-(5-Methoxypentoxy)-5,5,8,8-tetramethyl-5,6,7,8-tetrahydronaphth-2-yl]-3-hydroxyprop-1-ynyl}benzoic acid
16. 4-{3-[4-(6-Methoxyhexyloxy)-5,5,8,8-tetramethyl-5,6,7,8-tetrahydronaphth-2-yl]-3-hydroxyprop-1-ynyl}benzoic acid
17. 4-{3-[4-(2-Ethoxyethoxy)-5,5,8,8-tetramethyl-5,6,7,8-tetrahydronaphth-2-yl]-3-hydroxybut-1-ynyl}benzoic acid
18. 4-{3-[4-(2-Ethoxyethoxy)-5,5,8,8-tetramethyl-5,6,7,8-tetrahydronaphth-2-yl]-3-hydroxypent-1-ynyl}benzoic acid
19. 4-{3-[4-(2-Ethoxyethoxy)-5,5,8,8-tetramethyl-5,6,7,8-tetrahydronaphth-2-yl]-3-acetoxyprop-1-ynyl}benzoic acid
20. 4-{3-[4-(2-Ethoxyethoxy)-5,5,8,8-tetramethyl-5,6,7,8-tetrahydronaphth-2-yl]-3-propionyloxyprop-1-ynyl}benzoic acid
21. 4-{3-[7-(2-Ethoxyethoxy)-1,1,3,3-tetramethyl-1,3-dihydroisobenzofuran-5-yl]-3-hydroxyprop-1-ynyl}benzoic acid
22. 4-{3-[8-(2-Ethoxyethoxy)-4,4-dimethylchroman-6-yl]-3-hydroxyprop-1-ynyl}benzoic acid
23. 4-{3-[8-(2-Ethoxyethoxy)-4,4-dimethylthiochroman-6-yl]-3-hydroxyprop-1-ynyl}benzoic acid
24. 4-{3-[5-(2-Ethoxyethoxy)-4,4-dimethylchroman-7-yl]-3-hydroxyprop-1-ynyl}benzoic acid
25. 4-{3-[5-(2-Ethoxyethoxy)-4,4-dimethylthiochroman-7-yl]-3-hydroxyprop-1-ynyl}benzoic acid
26. 4-{3-[4-(2-Acetoxyethoxy)-5,5,8,8-tetramethyl-5,6,7,8-tetrahydronaphth-2-yl]-3-hydroxyprop-1-ynyl}benzoic acid
27. 4-(3-Hydroxy-3-{5,5,8,8-tetramethyl-4-[2-(2,2,2-trifluoroacetoxy)ethoxy]-5,6,7,8-tetrahydronaphth-2-yl}prop-1-ynyl)benzoic acid
28. 4-(3-Hydroxy-3-[5,5,8,8-tetramethyl-4-(2-propionyloxyethoxy)-5,6,7,8-tetrahydronaphth-2-yl]prop-1-ynyl}benzoic acid
29. 4-{3-[4-(2-Ethoxyethoxy)-5,5,8,8-tetramethyl-5,6,7,8-tetrahydronaphth-2-yl]-3-hydroxyprop-1-ynyl}-2-methylbenzoic acid
30. 2-Chloro-4-{3-[4-(2-ethoxyethoxy)-5,5,8,8-tetramethyl-5,6,7,8-tetrahydronaphth-2-yl]-3-hydroxyprop-1-ynyl}benzoic acid
31. 4-{3-[4-(2-Ethoxyethoxy)-5,5,8,8-tetramethyl-5,6,7,8-tetrahydronaphth-2-yl]-3-hydroxyprop-1-ynyl}-2-fluorobenzoic acid
32. 4-{3-[4-(2-Ethoxyethoxy)-5,5,8,8-tetramethyl-5,6,7,8-tetrahydronaphth-2-yl]-3-hydroxyprop-1-ynyl}-3-fluorobenzoic acid
33. 4-{3-[4-(2-Ethoxyethoxy)-5,5,8,8-tetramethyl-5,6,7,8-tetrahydronaphth-2-yl]-3-hydroxyprop-1-ynyl}-3-methylbenzoic acid
34. Ethyl 4-{3-[4-(2-ethoxyethoxy)-5,5,8,8-tetramethyl-5,6,7,8-tetrahydronaphth-2-yl]-3-hydroxyprop-1-ynyl}benzoate
35. Isopropyl 4-{3-[4-(2-ethoxyethoxy)-5,5,8,8-tetramethyl-5,6,7,8-tetrahydronaphth-2-yl]-3-hydroxyprop-1-ynyl}benzoate
36. Isobutyl 4-{3-[4-(2-ethoxyethoxy)-5,5,8,8-tetramethyl-5,6,7,8-tetrahydronaphth-2-yl]-3-hydroxyprop-1-ynyl}benzoate
37. 2,3-Dihydroxypropyl 4-{3-[4-(2-ethoxyethoxy)-5,5,8,8-tetramethyl-5,6,7,8-tetrahydronaphth-2-yl]-3-hydroxyprop-1-ynyl}benzoate
38. 6-Glucosyl 4-{3-[4-(2-ethoxyethoxy)-5,5,8,8-tetramethyl-5,6,7,8-tetrahydronaphth-2-yl]-3-hydroxyprop-1-ynyl}benzoate
39. 6-Galactosyl 4-{3-[4-(2-ethoxyethoxy)-5,5,8,8-tetramethyl-5,6,7,8-tetrahydronaphth-2-yl]-3-hydroxyprop-1-ynyl}benzoate
40. 4-{3-[4-(2-Ethoxyethoxy)-5,5,8,8-tetramethyl-5,6,7,8-tetrahydronaphth-2-yl]-3-hydroxyprop-1-ynyl}-N,N-dimethylbenzamide
41. 4-{3-[4-(2-Ethoxyethoxy)-5,5,8,8-tetramethyl-5,6,7,8-tetrahydronaphth-2-yl]-3-hydroxyprop-1-ynyl}-N,N-diethylbenzamide
42. (4-{3-[4-(2-Ethoxyethoxy)-5,5,8,8-tetramethyl-5,6,7,8-tetrahydronaphth-2-yl]-3-hydroxyprop-1-ynyl}phenyl)piperidin-1-ylmethanone
43. (4-{3-[4-(2-Ethoxyethoxy)-5,5,8,8-tetramethyl-5,6,7,8-tetrahydronaphth-2-yl]-3-hydroxyprop-1-ynyl}phenyl)morpholin-4-ylmethanone
44. (4-{3-[4-(2-Ethoxyethoxy)-5,5,8,8-tetramethyl-5,6,7,8-tetrahydronaphth-2-yl]-3-hydroxyprop-1-ynyl}phenyl)pyrrolidin-1-ylmethanone
45. (4-{3-[4-(2-Ethoxyethoxy)-5,5,8,8-tetramethyl-5,6,7,8-tetrahydronaphth-2-yl]-3-hydroxyprop-1-ynyl}-N-ethylbenzamide
46. (4-{3-[4-(2-Ethoxyethoxy)-5,5,8,8-tetramethyl-5,6,7,8-tetrahydronaphth-2-yl]-3-hydroxyprop-1-ynyl}benzoylamino)acetic acid
47. 2-(4-{3-[4-(2-Ethoxyethoxy)-5,5,8,8-tetramethyl-5,6,7,8-tetrahydronaphth-2-yl]-3-hydroxyprop-1-ynyl}benzoylamino)-3-phenylpropionic acid.

16. Compounds according to Claim 1 or 2, **characterized in that** they have one of the following characteristics:
Ar represents the radical (a),
n is 2 or 3,
R₂ represents a hydrogen atom,
R₄ and R₅ form, with the adjacent aromatic ring, a 6-membered ring,
R₆ represents an alkyl radical.

17. Compounds according to any one of Claims 1 to 16, as medicinal products.

18. Compounds according to any one of Claims 1 to 16 in the manufacture of a composition intended for treating:
- dermatological complaints associated with a keratinization disorder relating to cell differentiation and proliferation;
- ichthyosis, ichthyosiform conditions, Darier's disease, palmoplantar keratodermas, leukoplakia and leukoplakiform conditions, and cutaneous or mucous (buccal) lichen;
- dermatological complaints with an inflammatory immunoallergic component, with or without cell proliferation disorder;
- benign or malignant dermal or epidermal proliferations, of viral or non-viral origin;
- proliferations which may be induced by ultraviolet radiation;
- precancerous skin lesions;
- immune dermatoses;
- immune bullous diseases;
- collagen diseases;
- dermatological conditions with an immunological component;
- ophthalmological disorders;
- stigmata of epidermal and/or dermal atrophy induced by local or systemic corticosteroids, or any other form of cutaneous atrophy;
- skin complaints of viral origin;
- skin disorders caused by exposure to UV radiation, photoinduced or chronological ageing of the skin, or actinic pigmentations and keratoses;
- pathologies associated with chronological or actinic ageing of the skin;
- disorders of sebaceous function;
- cicatrization disorders or stretch marks; or
- pigmentation disorders.

19. Pharmaceutical composition, **characterized in that** it comprises, in a physiologically acceptable support, at least one of the compounds as defined in any one of Claims 1 to 16.

20. Composition according to Claim 19, **characterized in that** the concentration of compound(s) according to one of Claims 1 to 16 is between 0.001% and 10% by weight relative to the total weight of the composition.

21. Composition according to Claim 19, **characterized in that** the concentration of compound(s) according to one of Claims 1 to 16 is between 0.01% and 1% by weight relative to the total weight of the composition.

22. Cosmetic composition, **characterized in that** it comprises, in a physiologically acceptable medium, at least one of the compounds as defined in any one of Claims 1 to 16.

23. Composition according to Claim 22, **characterized in that** the concentration of compound(s) according to one of Claims 1 to 16 is between 0.001% and 3% by weight relative to the total weight of the composition.

24. Non-therapeutic use of a cosmetic composition as defined in either of Claims 22 and 23, for preventing and/or treating the signs of ageing and/or dry skin.

25. Non-therapeutic use of a cosmetic composition as defined in either of Claims 22 and 23, for body or hair hygiene.

26. Cosmetic process for enhancing the skin, **characterized in that** a composition as defined in either of Claims 22 and 23 is applied to the skin.

## Patentansprüche

1. Verbindungen, **dadurch gekennzeichnet, dass** sie der folgenden Formel (I) entsprechen: 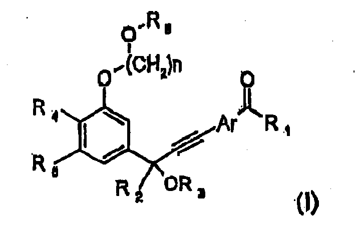 in der Formel:
- Ar ist eine Gruppe, die unter den Gruppen der folgenden Formeln (a) bis (c) ausgewählt ist: 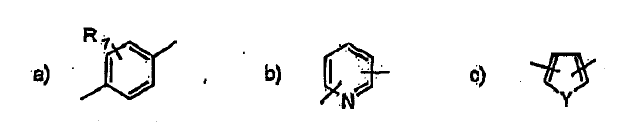 wobei R₇ und Y die nachstehend angegebenen Bedeutungen aufweisen;
- R₁ bedeutet:
(a) ein Wasserstoffatom,
(b) eine Alkylgruppe mit 1 bis 6 Kohlenstoffatomen,
(c) eine Gruppe der folgenden Formel: 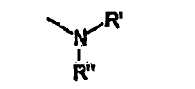
(d) eine Gruppe -OR₈,
wobei die Gruppen R', R" und R₈ die nachstehend angegebenen Bedeutungen aufweisen;
- R₂ bedeutet ein Wasserstoffatom oder eine Alkylgruppe mit 1 bis 6 Kohlenstoffatomen;
- R₃ bedeutet ein Wasserstoffatom, eine Alkylgruppe mit 1 bis 6 Kohlenstoffatomen oder eine Gruppe -CO-R₉,
wobei R₉ die nachstehend angegebene Bedeutung aufweist;
- R₄ und R₅ können gemeinsam mit dem angrenzenden aromatischen Ring einen 5- oder 6-gliedrigen Ring bilden, der gegebenenfalls mit Methylgruppen substituiert und/oder gegebenenfalls mit einem Sauerstoffatom oder einem Schwefelatom unterbrochen ist;
- R₆ bedeutet eine Alkylgruppe mit 1 bis 6 Kohlenstoffatomen oder eine Gruppe (CO)R₁₀,
wobei R₁₀ die nachstehend angegebene Bedeutung aufweist;
- R₇ bedeutet ein Wasserstoffatom, ein Halogenatom, eine Alkylgruppe mit 1 bis 6 Kohlenstoffatomen oder eine Gruppe -OR₁₁,
wobei R₁₁ die nachstehend angegebene Bedeutung aufweist;
- R₈ bedeutet ein Wasserstoffatom, eine lineare oder verzweigte Alkylgruppe mit 1 bis 20 Kohlenstoffatomen, eine Alkenylgruppe, eine Mono- oder Polyhydroxyalkylgruppe, eine gegebenenfalls substituierte Aryl- oder Aralkylgruppe oder einen Zuckerrest oder einen Aminosäurerest oder einen Peptidrest;
- R₉ und R₁₀ bedeuten CF₃ oder eine Alkylgruppe mit 1 bis 6 Kohlenstoffatomen;
- R₁₁ bedeutet H, eine Alkylgruppe mit 1 bis 6 Kohlenstoffatomen oder eine Gruppe (CO)R₁₂,
wobei R₁₂ die nachstehend angegebene Bedeutung aufweist;
- R₁₂ bedeutet eine Alkylgruppe mit 1 bis 6 Kohlenstoffatomen;
- R' und R" bedeuten ein Wasserstoffatom, eine Alkylgruppe mit 1 bis 6 Kohlenstoffatomen, eine Mono- oder Polyhydroxyalkylgruppe, eine gegebenenfalls substituierte Aroylgruppe oder einen Aminosäurerest, Peptidrest oder Zuckerrest oder sie bilden alternativ hierzu gemeinsam eine heterocyclische Gruppe;
- Y bedeutet ein Sauerstoff- oder Schwefelatom;
- n ist eine ganze Zahl im Bereich von 2 bis 6;
und die Salze der Verbindungen der Formel (I), wenn R₁ eine OH-Funktion bedeutet, und ferner die optischen und geometrischen Isomere der Verbindungen der Formel (I).

2. Verbindungen nach Anspruch 1, **dadurch gekennzeichnet, dass** sie in Form von Alkalimetallsalzen oder Erdalkalimetallsalzen, Zinksalzen oder Salzen eines organischen Amins vorliegen.

3. Verbindungen nach einem der Ansprüche 1 und 2, **dadurch gekennzeichnet, dass** die Alkylgruppen mit 1 bis 6 Kohlenstoffatomen unter den Gruppen Methyl, Ethyl, *n*-Propyl, *i*-Propyl, *c*-Propyl, Isopropyl, *n*-Butyl, *i*-Butyl, *t*-Butyl, *n*-Pentyl und *n*-Hexyl ausgewählt sind.

4. Verbindungen nach einem der Ansprüche 1 bis 3, **dadurch gekennzeichnet, dass** die Alkylgruppen mit 1 bis 20 Kohlenstoffatomen unter den Gruppen Methyl, Ethyl, *n*-Propyl, *i*-Propyl, *c*-Propyl, Isopropyl, Cyclopropyl, *n*-Butyl, *i*-Butyl, *t*-Butyl, *n*-Pentyl, *n*-Hexyl, 2-Ethylhexyl, Octyl, Dodecyl, Hexadecyl und Octadecyl ausgewählt sind.

5. Verbindungen nach einem der Ansprüche 1 bis 4, **dadurch gekennzeichnet, dass** die Monohydroxyalkylgruppen unter 2-Hydroxyethyl, 2-Hydroxypropyl und 3-Hydroxypropyl ausgewählt sind.

6. Verbindungen nach einem der Ansprüche 1 bis 5, **dadurch gekennzeichnet, dass** die Polyhydroxyalkylgruppen unter 2,3-Dihydroxypropyl, 2,3,4-Trihydroxybutyl oder 2,3,4,5-Tetrahydroxypentyl und dem Pentaerythritrest ausgewählt sind

7. Verbindungen nach einem der vorhergehenden Ansprüche, **dadurch gekennzeichnet, dass** die Arylgruppen unter den Phenylgruppen ausgewählt sind, die gegebenenfalls mit mindestens einem Halogen, einer Hydroxygruppe oder einer Nitrofunktion substituiert sind.

8. Verbindungen nach einem der vorhergehenden Ansprüche, **dadurch gekennzeichnet, dass** die Aralkylgruppen unter Benzyl und Phenethyl ausgewählt sind, wobei diese gegebenenfalls mit mindestens einem Halogen, einer Hydroxygruppe oder einer Nitrofunktion substituiert sind.

9. Verbindungen nach einem der vorhergehenden Ansprüche, **dadurch gekennzeichnet, dass** die Alkenylgruppen unter den Gruppen ausgewählt sind, die vorzugsweise 1 bis 6 Kohlenstoffatome aufweisen und eine oder mehrere ethylenisch ungesättigte Gruppen besitzen.
2. 4-{3-Hydroxy-3-[4-(2-ethoxyethoxy)-5,5,8,8-tetramethyl-5,6,7,8-tetrahydronaphth-2-yl]-prop-1-inyl}-benzoesäure
3. 4-{3-Hydroxy-3-[4-(2-ethoxyethoxy)-5,5,8,8-tetramethyl-5,6,7,8-tetrahydronaphth-2-yl]-prop-1-inyl}-2-hydroxybenzoesäure
4. 4-{3-Hydroxy-3-[4-(2-ethoxyethoxy)-5,5,8,8-tetramethyl-5,6,7,8-tetrahydronaphth-2-yl]-prop-1-inyl}-(S)-benzoesäure
5. 4-{3-Hydroxy-3-[4-(2-ethoxyethoxy)-5,5,8,8-tetramethyl-5,6,7,8-tetrahydronaphth-2-yl]-prop-1-inyl}-(R)-benzoesäure
6. 6-{3-Hydroxy-3-[4-(2-ethoxyethoxy)-5,5,8,8-tetramethyl-5,6,7,8-tetrahydronaphth-2-yl]-prop-1-inyl}-nicotinsäure
7. 5-{3-Hydroxy-3-[4-(2-ethoxyethoxy)-5,5,8,8-tetramethyl-5,6,7,8-tetrahydronaphth-2-yl]-prop-1-inyl}-pyridin-2-carbonsäure
8. 5-{3-[4-(2-Ethoxyethoxy)-5,5,8,8-tetramethyl-5,6,7,8-tetrahydronaphth-2-yl]-3-hydroxyprop-1-inyl}-thiophen-2-carbonsäure
9. 4-{3-[4-(2-Ethoxyethoxy)-5,5,8,8-tetramethyl-5,6,7,8-tetrahydronaphth-2-yl]-3-hydroxyprop-1-inyl}-thiophen-2-carbonsäure
10. 5-{3-[4-(2-Ethoxyethoxy)-5,5,8,8-tetramethyl-5,6,7,8-tetrahydronaphth-2-yl]-3-hydroxyprop-1-inyl}-thiophen-3-carbonsäure
11. 5-{3-[4-(2-Ethoxyethoxy)-5,5,8,8-tetramethyl-5,6,7,8-tetrahydronaphth-2-yl]-3-hydroxyprop-1-inyl}-furan-2-carbonsäure
12. 4-{3-[4-(3-Ethoxyethoxy)-5,5,8,8-tetramethyl-5,6,7,8-tetrahydronaphth-2-yl]-3-hydroxyprop-1-inyl}-benzoesäure
13. 4-{3-[4-(3-Methoxypropoxy)-5,5,8,8-tetramethyl-5,6,7,8-tetrahydronaphth-2-yl]-3-hydroxyprop-1-inyl}-benzoesäure

10. Verbindungen nach einem der vorhergehenden Ansprüche, **dadurch gekennzeichnet, dass** der Zuckerrest unter den von Glucose, Galactose oder Mannose oder alternativ Galacturonsäure abgeleiteten Resten ausgewählt ist.

11. Verbindungen nach einem der vorhergehenden Ansprüche, **dadurch gekennzeichnet, dass** der Aminosäurerest unter den Resten ausgewählt ist, die von Lysin, Glycin oder Asparaginsäure abgeleitet sind.

12. Verbindungen nach einem der vorhergehenden Ansprüche, **dadurch gekennzeichnet, dass** der Peptidrest unter den Dipeptidresten oder Tripeptidresten ausgewählt ist, die aus der Kombination von Aminosäuren resultieren.

13. Verbindungen nach einem der vorhergehenden Ansprüche, **dadurch gekennzeichnet, dass** die heterocyclischen Gruppen unter Morpholino, Pyrrolidino oder Piperazino ausgewählt sind, wobei sie gegebenenfalls in 4-Stellung mit einer C₁₋₆-Alkylgruppe oder einer Mono- oder Polyhydroxyalkylgruppe substituiert sind.

14. Verbindungen nach einem der vorhergehenden Ansprüche, **dadurch gekennzeichnet, dass** das Halogenatom unter Fluor, Chlor oder Brom ausgewählt ist.

15. Verbindungen der Formel (I) nach einem der vorhergehenden Ansprüche, die ausgewählt sind unter:
1. 4-{3-Hydroxy-3-[4-(2-methoxyethoxy)-5,5,8,8-tetramethyl-5,6,7,8-tetrahydronaphth-2-yl]-prop-1-inyl}-benzoesäure
14. 4-{3-[4-(4-Methoxybutoxy)-5,5,8,8-tetramethyl-5,6,7,8-tetrahydronaphth-2-yl]-3-hydroxyprop-1-inyl}-benzoesäure
15. 4-{3-[4-(5-Methoxypentoxy)-5,5,8,8-tetramethyl-5,6,7,8-tetrahydronaphth-2-yl]-3-hydroxyprop-1-inyl}-benzoesäure
16. 4-{3-[4-(6-Methoxyhexyloxy)-5,5,8,8-tetramethyl-5,6,7,8-tetrahydronaphth-2-yl]-3-hydroxyprop-1-inyl}-benzoesäure
17. 4-{3-[4-(2-Ethoxyethoxy)-5,5,8,8-tetramethyl-5,6,7,8-tetrahydronaphth-2-yl]-3-hydroxybut-1-inyl}-benzoesäure
18. 4-{3-[4-(2-Ethoxyethoxy)-5,5,8,8-tetramethyl-5,6,7,8-tetrahydronaphth-2-yl]-3-hydroxypent-1-inyl}-benzoesäure
19. 4-{3-[4-(2-Ethoxyethoxy)-5,5,8,8-tetramethyl-5,6,7,8-tetrahydronaphth-2-yl]-3-acetoxyprop-1-inyl}-benzoesäure
20. 4-{3-[4-(2-Ethoxyethoxy)-5,5,8,8-tetramethyl-5,6,7,8-tetrahydronaphth-2-yl]-3-propionyloxyprop-1-inyl}-benzoesäure
21. 4-{3-[7-(2-Ethoxyethoxy)-1,1,3,3-tetramethyl-1,3-dihydroisobenzofuran-5-yl]-3-hydroxyprop-1-inyl}-benzoesäure
22. 4-{3-[8-(2-Ethoxyethoxy)-4,4-dimethyl-chroman-6-yl]-3-hydroxyprop-1-inyl}-benzoesäure
23. 4-{3-[8-(2-Ethoxyethoxy)-4,4-dimethyl-thiochroman-6-yl]-3-hydroxyprop-1-inyl}-benzoesäure
24. 4-{3-[5-(2-Ethoxyethoxy)-4,4-dimethyl-chroman-7-yl]-3-hydroxyprop-1-inyl}-benzoesäure
25. 4-{3-[5-(2-Ethoxycthoxy)-4,4-dimethyl-thiochroman-7-yl]-3-hydroxyprop-1-inyl}-benzoesäure
26. 4-{3-[4-(2-Acetoxyethoxy)-5,5,8,8-tetramethyl-5,6,7,8-tetrahydronaphth-2-yl]-3-hydroxyprop-1-inyl}-benzoesäure
27. 4-{3-Hydroxy-3-{5,5,8,8-tetramethyl-4-[2-(2,2,2-trifluoracetoxy)ethoxy]-5,6,7,8-tetrahydronaphth-2-yl}-prop-1-inyl}-benzoesäure
28. 4-{3-Hydroxy-3-[5,5,8,8-tetramethyl-4-(2-propionyloxyethoxy)-5,6,7,8-tetrahydronaphth-2-yl]-prop-1-inyl}-benzoesäure
29. 4-{3-[4-(2-Ethoxyethoxy)-5,5,8,8-tetramethyl-5,6,7,8-tetrahydronaphth-2-yl]-3-hydroxyprop-1-inyl}-2-methylbenzoesäure
30. 2-Chlor-4-{3-[4-(2-ethoxyethoxy)-5,5,8,8-tetramethyl-5,6,7,8-tetrahydronaphth-2-yl]-3-hydroxyprop-1-inyl}-benzoesäure
31. 4-{3-[4-(2-Ethoxyethoxy)-5,5,8,8-tetramethyl-5,6,7,8-tetrahydronaphth-2-yl]-3-hydroxyprop-1-inyl}-2-fluorbenzoesäure
32. 4-{3-[4-(2-Ethoxyethoxy)-5,5,8,8-tetramethyl-5,6,7,8-tetrahydronaphth-2-yl]-3-hydroxyprop-1-inyl}-3-fluorbenzoesäure
33. 4-{3-[4-(2-Ethoxyethoxy)-5,5,8,8-tetramethyl-5,6,7,8-tetrahydronaphth-2-yl]-3-hydroxyprop-1-inyl}-3-methylbenzoesäure
34. Ethyl-4-{3-[4-(2-ethoxyethoxy)-5,5,8,8-tetramethyl-5,6,7,8-tetrahydronaphth-2-yl]-3-hydroxyprop-1-inyl}-benzoat
35. Isopropyl-4-{3-[4-(2-ethoxyethoxy)-5,5,8,8-tetramethyl-5,6,7,8-tetrahydronaphth-2-yl]-3-hydroxyprop-1-inyl}-benzoat
36. Isobutyl-4-{3-[4-(2-ethoxyethoxy)-5,5,8,8-tetramethyl-5,6,7,8-tetrahydronaphth-2-yl]-3-hydroxyprop-1-inyl}-benzoat
37. 2,3-Dihydroxypropyl-4-{3-[4-(2-ethoxyethoxy)-5,5,8,8-tetramethyl-5,6,7,8-tetrahydronaphth-2-yl]-3-hydroxyprop-1-inyl}-benzoat
38. 6-Glucosyl-4-{3-[4-(2-ethoxyethoxy)-5,5,8,8-tetramethyl-5,6,7,8-tetrahydronaphth-2-yl]-3-hydroxyprop-1-inyl}-benzoat
39. 6-Galactosyl-4-{3-[4-(2-ethoxyethoxy)-5,5,8,8-tetramethyl-5,6,7,8-tetrahydronaphth-2-yl]-3-hydroxyprop-1-inyl}-benzoat
40. 4-{3-[4-(2-Ethoxyethoxy)-5,5,8,8-tetramethyl-5,6,7,8-tetrahydronaphth-2-yl]-3-hydroxyprop-1-inyl}-N,N-dimethylbenzamid
41. 4-{3-[4-(2-Ethoxyethoxy)-5,5,8,8-tetramethyl-5,6,7,8-tetrahydronaphth-2-yl]-3-hydroxyprop-1-inyl}-N,N-diethylbenzamid
42. (4-{3-[4-(2-Ethoxyethoxy)-5,5,8,8-tetramethyl-5,6,7,8-tetrahydronaphth-2-yl]-3-hydroxyprop-1-inyl}phenyl)-piperidin-1-ylmethanon
43. (4-{3-[4-(2-Ethoxyethoxy)-5,5,8,8-tetramethyl-5,6,7,8-tetrahydronaphth-2-yl]-3-hydroxyprop-1-inyl}phenyl)-morpholin-4-ylmethanon
44. (4-{3-[4-(2-Ethoxyethoxy)-5,5,8,8-tetramethyl-5,6,7,8-tetrahydronaphth-2-yl]-3-hydroxyprop-1-inyl}phenyl)-pyrrolidin-1-ylmethanon
45. 4-{3-[4-(2-Ethoxyethoxy)-5,5,8,8-tetramethyl-5,6,7,8-tetrahydronaphth-2-yl]-3-hydroxyprop-1-inyl}-N-ethylbenzamid
46. (4-{3-[4-(2-Ethoxyethoxy)-5,5,8,8-tetramethyl-5,6,7,8-tetrahydronaphth-2-yl]-3-hydroxyprop-1-inyl}-benzoylamino)-essigsäure
47. 2-(4-{3-[4-(2-Ethoxyethoxy)-5,5,8,8-tetramethyl-5,6,7,8-tetrahydronaphth-2-yl]-3-hydroxyprop-1-inyl}-benzoylamino)-3-phenylpropionsäure.

16. Verbindungen nach Anspruch 1 oder 2, **dadurch gekennzeichnet, dass** sie eines der folgenden Merkmale aufweisen:
A bedeutet die Gruppe (a),
n ist 2 oder 3,
R₂ bedeutet ein Wasserstoffatom,
R₄ und R₅ bilden mit dem angrenzenden aromatischen Ring einen 6-gliedrigen Ring,
R₆ bedeutet eine Alkylgruppe.

17. Verbindungen nach einem der Ansprüche 1 bis 16 als Arzneimittel.

18. Verbindungen nach einem der Ansprüche 1 bis 16 für die Herstellung einer Zusammensetzung, die vorgesehen ist für die Behandlung von:
- dermatologischen Erkrankungen, die mit einer Verhornungsstörung verbunden sind, die auf der Differenzierung und Proliferation der Zellen beruht;
- Ichtyosis, ichtyosisartigen Zuständen, der Darier Krankheit, Palmoplantarkeratosen, Leukoplakien und leucoplakieformen Zuständen und Lichen der Haut oder der Schleimhäute (buccal);
- dermatologischen Erkrankungen mit entzündlicher immunoallergischer Komponente, mit oder ohne Störung der Proliferation der Zellen;
- Proliferationen der Dermis oder Epidermis, die gutartig oder bösartig und gegebenenfalls viralen Ursprungs sein können;
- Proliferationen, die von UV-Strahlung induziert sein können;
- präkanzerösen Hautläsionen;
- Immundermatosen;
- bullösen Immunerkrankungen;
- Kollagenerkrankungen;
- dermatologischen Erkrankungen mit immunologischer Komponente;
- ophthalmologischen Erkrankungen;
- Stigmen epidermaler und/oder dermaler Atrophie, die durch lokale oder systemische Corticosteroide hervorgerufen werden, oder aller anderen Formen der Atrophie der Haut;
- Hauterkrankungen viralen Ursprungs;
- Hautstörungen, die mit einer UV-Exposition zusammenhängen; altersbedingter oder lichtinduzierter Hautalterung, aktinischen Pigmentierungen oder Keratosen;
- Pathologien, die mit der altersbedingten oder aktinischen Hautalterung einhergehen;
- Störungen der Talgdrüsenfunktion;
- Störungen der Wundheilung oder Streifen; oder
- Pigmentierungsstörungen.

19. Pharmazeutische Zusammensetzung, **dadurch gekennzeichnet, dass** sie in einem pharmazeutisch akzeptablen Träger mindestens eine Verbindung enthält, wie sie in einem der Ansprüche 1 bis 16 definiert ist.

20. Zusammensetzung nach Anspruch 19, **dadurch gekennzeichnet, dass** die Konzentration der Verbindung(en) nach einem der Ansprüche 1 bis 16 im Bereich von 0,001 bis 10 Gew.-%, bezogen auf das Gesamtgewicht der Zusammensetzung, liegt.

21. Zusammensetzung nach Anspruch 19, **dadurch gekennzeichnet, dass** die Konzentration der Verbindung(en) nach einem der Ansprüche 1 bis 16 im Bereich von 0,01 bis 1 Gew.-%, bezogen auf das Gesamtgewicht der Zusammensetzung, liegt.

22. Kosmetische Zusammensetzung, **dadurch gekennzeichnet, dass** sie in einem physiologisch akzeptablen Medium mindestens eine Verbindung enthält, wie sie in einem der Ansprüche 1 bis 16 definiert ist.

23. Zusammensetzung nach Anspruch 22, **dadurch gekennzeichnet, dass** die Konzentration der Verbindung(en) nach einem der Ansprüche 1 bis 16 im Bereich von 0,001 bis 3 Gew.-%, bezogen auf das Gesamtgewicht der Zusammensetzung, liegt.

24. Nichttherapeutische Verwendung einer kosmetischen Zusammensetzung, wie sie in Anspruch 22 oder 23 definiert ist, um den Anzeichen von Alterung und/oder trockener Haut vorzubeugen und/ oder diese zu behandeln.

25. Nichttherapeutische Verwendung einer kosmetischen Zusammensetzung, wie sie in Anspruch 22 oder 23 definiert ist, für die Körperpflege oder Pflege des Haares.

26. Kosmetisches Verfahren zur Verschönerung der Haut, **dadurch gekennzeichnet, dass** eine Zusammensetzung, wie sie in Anspruch 22 oder 23 definiert ist, auf die Haut aufgetragen wird.

## Revendications

1. Composés **caractérisés en ce qu'**ils correspondent à la formule (I) ci-dessous : 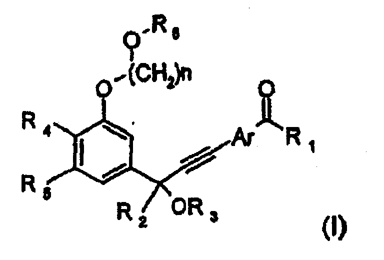 dans laquelle :
Ar représente un radical choisi parmi les radicaux de formules (a)-(c) ci-dessous : 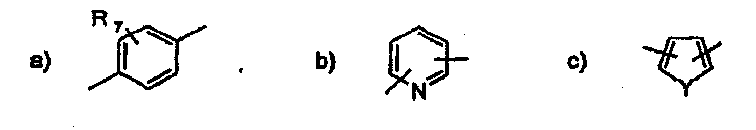
R₇ et Y présentant les significations indiquées ci-dessous,
- R₁ représente un radical :
(a) un atome d'hydrogène,
(b) un radical alkyle renfermant de 1 à 6 atomes de carbone,
(c) un radical de formule : 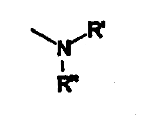
(d) un radical -OR₈ ;
R, R'' et R₈ présentant les significations indiquées ci-dessous,
- R₂ représente un atome d'hydrogène ou un radical alkyle renfermant de 1 à 6 atomes de carbone,
- R₃ représente un atome d'hydrogène, un radical alkyle renfermant de 1 à 6 atomes de carbone ou un radical -CO-R₉ ;
R₉ présentant la signification indiquée ci-dessous,
- R₄ et R₅, pris conjointement, peuvent former, avec le noyau aromatique adjacent, un noyau à 5 ou 6 chaînons, éventuellement substitué par des groupes méthyle et/ou éventuellement interrompu par un atome d'oxygène ou de soufre,
- R₆ représente un radical alkyle renfermant de 1 à 6 atomes de carbone ou un radical (CO)R₁₀ ;
R₁₀ présentant les significations indiquées ci-dessous,
- R₇ représente un atome d'hydrogène, un atome d'halogène, un radical alkyle renfermant de 1 à 6 atomes de carbone ou un radical -OR₁₁,
R₁₁ présentant la signification indiquée ci-dessous,
- R₈ représente un atome d'hydrogène, un radical alkyle linéaire ou ramifié renfermant de 1 à 20 atomes de carbone, un radical alcényle, un radical mono- ou polyhydroxyalkyle, un radical aryle ou aralkyle éventuellement substitué, ou un résidu glucidique ou un résidu d'acide aminé ou peptidique,
- R₉ et R₁₀ représentent un groupe CF₃ ou un radical alkyle renfermant de 1 à 6 atomes de carbone ;
- R₁₁ représente un atome d'hydrogène, un radical alkyle renfermant de 1 à 6 atomes de carbone ou un radical (CO)-R₁₂ ;
R₁₂ présentant la signification indiquée ci-dessous,
- R₁₂ représente un radical alkyle renfermant de 1 à 6 atomes de carbone ;
- R' et R'' représentent un atome d'hydrogène, un radical alkyle renfermant de 1 à 6 atomes de carbone, un radical mono- ou polyhydroxyalkyle, un radical aryle éventuellement substitué ou un résidu d'acide aminé, peptidique ou glucidique, ou en variante, pris conjointement, forment un hétérocycle,
- Y représente un atome d'oxygène ou de soufre ;
- n est un entier compris entre 2 et 6,
et les sels des composés de formule (I) dans laquelle R₁ représente une fonction OH, ainsi que les isomères optiques et géométriques desdits composés de formule (I).

2. Composés selon la revendication 1, **caractérisés en ce qu'**ils sont sous la forme de sels d'un métal alcalin ou d'un métal alcalino-terreux, de sels de zinc, ou de sels d'une amine organique.

3. Composés selon l'une ou l'autre des revendications 1 et 2, **caractérisés en ce que** les radicaux alkyle renfermant de 1 à 6 atomes de carbone sont choisis parmi les radicaux méthyle, éthyle, n-propyle, i-propyle, c-propyle, isopropyle, n-butyle, i-butyle, t-butyle, n-pentyle et n-hexyle.

4. Composés selon l'une des revendications 1 à 3, **caractérisés en ce que** les radicaux alkyle renfermant de 1 à 20 atomes de carbone sont choisis parmi les radicaux méthyle, éthyle, n-propyle, i-propyle, c-propyle, isopropyle, cyclopropyle, n-butyle, i-butyle, t-butyle, n-pentyle, n-hexyle, 2-éthylhexyle, octyle, dodécyle, hexadécyle et octadécyle.

5. Composés selon l'une des revendications 1 à 4, **caractérisés en ce que** les radicaux monohydroxyalkyle sont choisis parmi les radicaux 2-hydroxyéthyle, 2-hydroxypropyle et 3-hydroxypropyle.

6. Composés selon l'une des revendications 1 à 5, **caractérisés en ce que** les radicaux polyhydroxyalkyle sont choisis parmi les radicaux 2,3-dihydroxypropyle, 2,3,4-trihydroxybutyle ou 2,3,4,5-tétrahydroxypentyle et un résidu pentaérythritol.

7. Composés selon l'une quelconque des revendications précédentes, **caractérisés en ce que** les radicaux aryle sont choisis parmi des radicaux phényle éventuellement substitués par au moins un atome d'halogène, une fonction hydroxyle ou une fonction nitro.

8. Composés selon l'une quelconque des revendications précédentes, **caractérisés en ce que** les radicaux aralkyle sont choisis parmi des radicaux benzyle et phénéthyle, éventuellement substitués par au moins un atome d'halogène, une fonction hydroxyle ou une fonction nitro.

9. Composés selon l'une quelconque des revendications précédentes, **caractérisés en ce que** les radicaux alcényle sont choisis parmi des radicaux renfermant de préférence de 1 à 6 atomes de carbone et renfermant une ou plusieurs insaturations éthyléniques.

10. Composés selon l'une quelconque des revendications précédentes, **caractérisés en ce que** le résidu glucidique est choisi parmi le groupe constitué du résidu dérivé du glucose, du galactose ou du mannose, ou en variante de l'acide glucuronique.

11. Composés selon l'une quelconque des revendications précédentes, **caractérisés en ce que** le résidu d'acide aminé est choisi parmi le groupe constitué d'un résidu dérivé de la lysine, de la glycine ou de l'acide aspartique.

12. Composés selon l'une quelconque des revendications précédentes, **caractérisés en ce que** le résidu peptidique est choisi parmi le groupe constitué d'un résidu dipeptidique ou tripeptidique résultant de la combinaison d'acides aminés.

13. Composés selon l'une quelconque des revendications précédentes, **caractérisés en ce que** les radicaux hétérocycliques sont choisis parmi des radicaux morpholino, pyrrolidino et pipérazino, éventuellement substitués en position 4 par un radical alkyle en C₁-C₆ ou un radical mono- ou polyhydroxyalkyle.

14. Composés selon l'une quelconque des revendications précédentes, **caractérisés en ce que** les atomes d'halogène sont choisis parmi des atomes de fluor, de chlore et de brome.

15. Composés de formule (I) selon l'une quelconque des revendications précédentes, choisis parmi :
1. L'acide 4-{3-hydroxy-3-[4-(2-méthoxyéthoxy)-5,5,8,8-tétraméthyl-5,6,7,8-tétrahydronapht-2-yl]prop-1-ynyl}benzoïque
2. L'acide 4-{3-hydroxy-3-[4-(2-éthoxyéthoxy)-5,5,8,8-tétraméthyl-5,6,7,8-tétrahydronapht-2-yl]prop-1-ynyl}benzoïque
3. L'acide 4-{3-hydroxy-3-[4-(2-éthoxyéthoxy)-5,5,8,8-tétraméthyl-5,6,7,8-tétrahydronapht-2-yl]prop-1-ynyl}-2-hydroxybenzoïque
4. Le (S)-acide 4-{3-hydroxy-3-[4-(2-éthoxyéthoxy)-5,5,8,8-tétraméthyl-5,6,7,8-tétrahydronapht-2-yl]prop-1-ynyl}benzoïque
5. Le (R)-acide 4-{3-hydroxy-3-[4-(2-éthoxyéthoxy)-5,5,8,8-tétraméthyl-5,6,7,8-tétrahydronapht-2-yl]prop-1-ynyl}benzoïque
6. L'acide 6-{3-hydroxy-3-[4-(2-éthoxyéthoxy)-5,5,8,8-tétraméthyl-5,6,7,8-tétrahydronapht-2-yl]prop-1-ynyl}nicotinique
7. L'acide 5-{3-hydroxy-3-[4-(2-éthoxyéthoxy)-5,5,8,8-tétraméthyl-5,6,7,8-tétrahydronapht-2-yl]prop-1-ynyl}pyridine-2-carboxylique
8. L'acide 5-{3-[4-(2-éthoxyéthoxy)-5,5,8,8-tétraméthyl-5,6,7,8-tétrahydronapht-2-yl]-3-hydroxyprop-1-ynyl}thiophène-2-carboxylique
9. L'acide 4-{3-[4-(2-éthoxyéthoxy)-5,5,8,8-tétraméthyl-5,6,7,8-tétrahydronapht-2-yl]-3-hydroxyprop-1-ynyl}thiophène-2-carboxylique
10. L'acide 5-{3-[4-(2-éthoxyéthoxy)-5,5,8,8-tétraméthyl-5,6,7,8-tétrahydronapht-2-yl]-3-hydroxyprop-1-ynyl}thiophène-3-carboxylique
11. L'acide 5-{3-[4-(2-éthoxyéthoxy)-5,5,8,8-tétraméthyl-5,6,7,8-tétrahydronapht-2-yl]-3-hydroxyprop-1-ynyl}furane-2-carboxylique
12. L'acide 4-{3-[4-(3-éthoxyéthoxy)-5,5,8,8-tétraméthyl-5,6,7,8-tétrahydronapht-2-yl]-3-hydroxyprop-1-ynyl}benzoïque
13. L'acide 4-{3-[4-(3-méthoxypropoxy)-5,5,8,8-tétraméthyl-5,6,7,8-tétrahydronapht-2-yl]-3-hydroxyprop-1-ynyl}benzoïque
14. L'acide 4-{3-[4-(4-méthoxybutoxy)-5,5,8,8-tétraméthyl-5,6,7,8-tétrahydronapht-2-yl]-3-hydroxyprop-1-ynyl}benzoïque
15. L'acide 4-{3-[4-(5-méthoxypentoxy)-5,5,8,8-tétraméthyl-5,6,7,8-tétrahydronapht-2-yl]-3-hydroxyprop-1-ynyl}benzoïque
16. L'acide 4-{3-[4-(6-méthoxyhexyloxy)-5,5,8,8-tétraméthyl-5,6,7,8-tétrahydronapht-2-yl]-3-hydroxyprop-1-ynyl}benzoïque
17. L'acide 4-{3-[4-(2-éthoxyéthoxy)-5,5,8,8-tétraméthyl-5,6,7,8-tétrahydronapht-2-yl]-3-hydroxybut-1-ynyl}benzoïque
18. L'acide 4-{3-[4-(2-éthoxyéthoxy)-5,5,8,8-tétraméthyl-5,6,7,8-tétrahydronapht-2-yl]-3-hydroxypent-1-ynyl}benzoïque
19. L'acide 4-{3-[4-(2-éthoxyéthoxy)-5,5,8,8-tétraméthyl-5,6,7,8-tétrahydronapht-2-yl]-3-acétoxyprop-1-ynyl}benzoïque
20. L'acide 4-{3-[4-(2-éthoxyéthoxy)-5,5,8,8-tétraméthyl-5,6,7,8-tétrahydronapht-2-yl]-3-propionyloxyprop-1-ynyl}benzoïque
21. L'acide 4-{3-[7-(2-éthoxyéthoxy)-1,1,3,3-tétraméthyl-1,3-dihydroisobenzofuran-5-yl]-3-hydroxyprop-1-ynyl}benzoïque
22. L'acide 4-{3-[8-(2-éthoxyéthoxy)-4,4-diméthylchroman-6-yl]-3-hydroxyprop-1-ynyl}benzoïque
23. L'acide 4-{3-[8-(2-éthoxyéthoxy)-4,4-diméthylthiochroman-6-yl]-3-hydroxyprop-1-ynyl}benzoïque
24. L'acide 4-{3-[5-(2-éthoxyéthoxy)-4,4-diméthylchroman-7-yl]-3-hydroxyprop-1-ynyl}benzoïque
25. L'acide 4-{3-[5-(2-éthoxyéthoxy)-4,4-diméthylthiochroman-7-yl]-3-hydroxyprop-1-ynyl}benzoïque
26. L'acide 4-{3-[4-(2-acétoxyéthoxy)-5,5,8,8-tétraméthyl-5,6,7,8-tétrahydronapht-2-yl]-3-hydroxyprop-1-ynyl}benzoïque
27. L'acide 4-(3-hydroxy-3-{5,5,8,8-tétraméthyl-4-[2-(2,2,2-trifluoroacétoxy)éthoxy]-5,6,7,8-tétrahydronapht-2-yl}prop-1-ynyl)benzoïque
28. L'acide 4-(3-hydroxy-3-[5,5,8,8-tétraméthyl-4-(2-propionyloxyéthoxy)-5,6,7,8-tétrahydronapht-2-yl]prop-1-ynyl)benzoïque
29. L'acide 4-{3-[4-(2-éthoxyéthoxy)-5,5,8,8-tétraméthyl-5,6,7,8-tétrahydronapht-2-yl]-3-hydroxyprop-1-ynyl}-2-méthylbenzoïque
30. L'acide 2-chloro-4-{3-[4-(2-éthoxyéthoxy)-5,5,8,8-tétraméthyl-5,6,7,8-tétrahydronapht-2-yl]-3-hydroxyprop-1-ynyl}benzoïque
31. L'acide 4-{3-[4-(2-éthoxyéthoxy)-5,5,8,8-tétraméthyl-5,6,7,8-tétrahydronapht-2-yl]-3-hydroxyprop-1-ynyl}-2-fluorobenzoïque
32. L'acide 4-{3-[4-(2-éthoxyéthoxy)-5,5,8,8-tétraméthyl-5,6,7,8-tétrahydronapht-2-yl]-3-hydroxyprop-1-ynyl}-3-fluorobenzoïque
33. L'acide 4-{3-[4-(2-éthoxyéthoxy)-5,5,8,8-tétraméthyl-5,6,7,8-tétrahydronapht-2-yl]-3-hydroxyprop-1-ynyl}-3-méthylbenzoïque
34. Le 4-{3-[4-(2-éthoxyéthoxy)-5,5,8,8-tétraméthyl-5,5,7,8-tétrahydronapht-2-yl]-3-hydroxyprop-1-ynyl}benzoate d'éthyle
35. Le 4-{3-[4-(2-éthoxyéthoxy)-5,5,8,8-tétraméthyl-5,6,7,8-tétrahydronapht-2-yl]-3-hydroxyprop-1-ynyl}benzoate d'isopropyle
36. Le 4-{3-[4-(2-éthoxyéthoxy)-5,5,8,8-tétraméthyl-5,6,7,8-tétrahydronapht-2-yl]-3-hydroxyprop-1-ynyl}benzoate d'isobutyle
37. Le 4-{3-[4-(2-éthoxyéthoxy)-5,5,8,8-tétraméthyl-5,6,7,8-tétrahydronapht-2-yl]-3-hydroxyprop-1-ynyl}bénzoate de 2,3-dihydroxypropyle
38. Le 4-{3-[4-(2-éthoxyéthoxy)-5,5,8,8-tétraméthyl-5,6,7,8-tétrahydronapht-2-yl]-3-hydroxyprop-1-ynyl}benzoate de 6-glucosyle
39. Le 4-{3-[4-(2-éthoxyéthoxy)-5,5,8,8-tétraméthyl-5,6,7,8-tétrahydronapht-2-yl]-3-hydroxyprop-1-ynyl}benzoate de 6-galactosyle
40. Le 4-{3-[4-(2-éthoxyéthoxy)-5,5,8,8-tétraméthyl-5,6,7,8-tétrahydronapht-2-yl]-3-hydroxyprop-1-ynyl}-N,N-diméthylbenzamide
41. Le 4-{3-[4-(2-éthoxyéthoxy)-5,5,8,8-tétraméthyl-5,6,7,8-tétrahydronapht-2-yl]-3-hydroxyprop-1-ynyl}-N,N-diéthylbenzamide
42. La (4-{3-[4-(2-éthoxyéthoxy)-5,5,8,8-tétraméthyl-5,6,7,8-tétrahydronapht-2-yl]-3-hydroxyprop-1-ynyl}phényl)pipéridin-1-ylméthanone
43. La (4-{3-[4-(2-éthoxyéthoxy)-5,5,8,8-tétraméthyl-5,6,7,8-tétrahydronapht-2-yl]-3-hydroxyprop-1-ynyl}phényl)morpholin-4-ylméthanone
44. La (4-{3-[4-(2-éthoxyéthoxy)-5,5,8,8-tétraméthyl-5,6,7,8-tétrahydronapht-2-yl]-3-hydroxyprop-1-ynyl}phényl)pyrrolidin-1-ylméthanone
45. Le (4-{3-[4-(2-éthoxyéthoxy)-5,5,8,8-tétraméthyl-5,6,7,8-tétrahydronapht-2-yl]-3-hydroxyprop-1-ynyl}-N-éthylbenzamide
46. L'acide (4-{3-[4-(2-éthoxyéthoxy)-5,5,8,8-tétraméthyl-5,6,7,8-tétrahydronapht-2-yl]-3-hydroxyprop-1-ynyl}benzoylamino)acétique
47. L'acide 2-(4-{3-[4-(2-éthoxyéthoxy)-5,5,8,8-tétraméthyl-5,6,7,8-tétrahydronapht-2-yl]-3-hydroxyprop-1-ynyl}benzoylamino)-3-phénylpropionique

16. Composés selon la revendication 1 ou 2, **caractérisés en ce qu'**ils présentent l'une des caractéristiques suivantes :
Ar représente le radical (a),
n vaut 2 ou 3,
R₂ représente un atome d'hydrogène,
R₄ et R₅ forment, avec le noyau aromatique adjacent, un noyau à 6 chaînons,
R₆ représente un radical alkyle.

17. Composés selon l'une quelconque des revendications 1 à 16, en tant que produits médicinaux.

18. Composés selon l'une quelconque des revendications 1 à 16, pour la fabrication d'une composition destinée à traiter :
- des affections dermatologiques associées à un trouble de kératinisation lié à la différenciation et à la prolifération cellulaires ;
- une ichtyose, des pathologies ichtyosiformes, la maladie de Darier, des kératodermies palmo-plantaires, une leucoplasie et des pathologies leucoplasiformes, et un lichen cutané ou muqueux (buccal) ;
- des affections dermatologiques avec une composante immuno-allergique inflammatoire, avec ou sans trouble de prolifération cellulaire ;
- des proliférations dermiques ou épidermiques, bénignes ou malignes, d'origine virale ou non virale ;
- des proliférations qui peuvent être induites par le rayonnement ultraviolet ;
- des lésions cutanées pré-cancéreuses ;
- des dermatoses immunes ;
- des maladies bulleuses immunes ;
- des maladies du collagène ;
- des pathologies dermatologiques avec une composante immunologique ;
- des troubles ophtalmologiques ;
- des stigmates d'une atrophie épidermique et/ou dermique induite par des corticostéroïdes locaux ou systémiques, ou toute autre forme d'atrophie cutanée ;
- des affections cutanées d'origine virale ;
- des troubles cutanés causés par une exposition au rayonnement UV, un vieillissement photo-induit ou chronologique de la peau, ou des pigmentations actiniques et des kératoses ;
- des pathologies associées à un vieillissement chronologique ou actinique de la peau ;
- des troubles de la fonction sébacée ;
- des troubles de cicatrisation ou des vergetures ; ou
- des troubles de pigmentation.

19. Composition pharmaceutique, **caractérisée en ce qu'**elle comprend, dans un support physiologiquement acceptable, au moins l'un des composés tels que définis dans l'une quelconque des revendications 1 à 16.

20. Composition selon la revendication 19, **caractérisée en ce que** la concentration de composé(s) selon l'une des revendications 1 à 16 est comprise entre 0,001 % et 10 % en poids, par rapport au poids total de la composition.

21. Composition selon la revendication 19, **caractérisée en ce que** la concentration de composé(s) selon l'une des revendications 1 à 16 est comprise entre 0,01 % et 1 % en poids, par rapport au poids total de la composition.

22. Composition cosmétique, **caractérisée en ce qu'**elle comprend, dans un milieu physiologiquement acceptable, au moins l'un des composés tels que définis dans l'une quelconque des revendications 1 à 16.

23. Composition selon la revendication 22, **caractérisée en ce que** la concentration de composé(s) selon l'une des revendications 1 à 16 est comprise entre 0,001 % et 3 % en poids, par rapport au poids total de la composition.

24. Utilisation non thérapeutique d'une composition cosmétique telle que définie dans l'une ou l'autre des revendications 22 et 23, destinée à la prévention et/ou au traitement des signes d'un vieillissement et/ou d'une peau sèche.

25. Utilisation non thérapeutique d'une composition cosmétique telle que définie dans l'une ou l'autre des revendications 22 et 23, pour l'hygiène du corps ou des cheveux.

26. Procédé cosmétique pour améliorer la peau, **caractérisé en ce qu'**une composition telle que définie dans l'une ou l'autre des revendications 22 et 23 est appliquée sur la peau.
